(19) Europäisches Patentamt
European Patent Office
Office européen des brevets

(11) EP 3 389 857 B1

(12) # FASCICULE DE BREVET EUROPEEN

(45) Date de publication et mention de la délivrance du brevet:
**27.11.2019 Bulletin 2019/48**

(21) Numéro de dépôt: **16825519.8**

(22) Date de dépôt: **15.12.2016**

(51) Int Cl.:
***B01J 27/22*** $^{(2006.01)}$ ***B01J 23/755*** $^{(2006.01)}$
***B01J 23/89*** $^{(2006.01)}$ ***B01J 35/00*** $^{(2006.01)}$
***B01J 37/08*** $^{(2006.01)}$ ***C07C 1/04*** $^{(2006.01)}$
***C07C 9/04*** $^{(2006.01)}$ ***C10G 2/00*** $^{(2006.01)}$
***A61N 2/00*** $^{(2006.01)}$ ***B01J 23/745*** $^{(2006.01)}$

(86) Numéro de dépôt international:
**PCT/FR2016/053451**

(87) Numéro de publication internationale:
**WO 2017/103492 (22.06.2017 Gazette 2017/25)**

(54) **NANOPARTICULES DE CARBURE DE FER, PROCÉDÉ POUR LEUR PRÉPARATION ET LEUR UTILISATION POUR LA PRODUCTION DE CHALEUR**

EISENCARBIDNANOPARTIKEL, VERFAHREN ZUR HERSTELLUNG DAVON UND VERWENDUNG DAVON ZUR WÄRMEERZEUGUNG

IRON CARBIDE NANOPARTICLES, METHOD FOR PREPARING SAME AND USE THEREOF FOR HEAT GENERATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.12.2015 FR 1562763**

(43) Date de publication de la demande:
**24.10.2018 Bulletin 2018/43**

(73) Titulaires:
- **Institut National des Sciences Appliquées de Toulouse
31400 Toulouse (FR)**
- **Centre National de la Recherche Scientifique
75016 Paris (FR)**

(72) Inventeurs:
- **BORDET, Alexis
45470 Mülheim an der Ruhr (DE)**
- **SOULANTIKA, Aikaterini
31810 Clermont Le Fort (FR)**
- **CHAUDRET, Bruno
31320 Vigoulet Auzil (FR)**

(74) Mandataire: **Ipside
6, Impasse Michel Labrousse
31100 Toulouse (FR)**

(56) Documents cités:
**FR-A1- 3 003 774**

- **ANCA MEFFRE ET AL: "A Simple Chemical Route toward Monodisperse Iron Carbide Nanoparticles Displaying Tunable Magnetic and Unprecedented Hyperthermia Properties", NANO LETTERS, vol. 12, no. 9, 30 juillet 2012 (2012-07-30), pages 4722-4728, XP055305794, US ISSN: 1530-6984, DOI: 10.1021/nl302160d cité dans la demande**
- **VINCIANE KELSEN ET AL: "The use of ultrasmall iron(0) nanoparticles as catalysts for the selective hydrogenation of unsaturated C-C bonds", CHEMICAL COMMUNICATIONS, vol. 49, no. 33, 5 mars 2013 (2013-03-05), page 3416, XP055088989, ISSN: 1359-7345, DOI: 10.1039/c3cc00152k**
- **SASCHA CEYLAN ET AL: "Inductive Heating for Organic Synthesis by Using Functionalized Magnetic Nanoparticles Inside Microreactors", ANGEWANDTE CHEMIE INTERNATIONAL EDITION, vol. 47, no. 46, 3 novembre 2008 (2008-11-03), pages 8950-8953, XP055089006, ISSN: 1433-7851, DOI: 10.1002/anie.200801474**
- **LUO M ET AL: "Fischer-Tropsch Synthesis", CATALYSIS TODAY, ELSEVIER, NL, vol. 140, no. 3-4, 28 février 2009 (2009-02-28), pages 127-134, XP025879872, ISSN: 0920-5861 [extrait le 2008-11-22]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 389 857 B1

## Description

La présente invention s'inscrit dans le domaine des nanoparticules ferromagnétiques. Plus particulièrement, elle concerne des nanoparticules de carbure de fer, ainsi qu'un procédé de préparation de telles nanoparticules. L'invention concerne également l'utilisation de telles nanoparticules pour la production de chaleur, ainsi que pour la catalyse de réactions chimiques, en particulier pour la catalyse de la réaction de réduction du dioxyde de carbone ou du monoxyde de carbone en hydrocarbure(s).

On entend dans la présente description, par nanoparticules, des particules de taille comprise entre environ 1 nm et environ 100 nm.

Les nanoparticules magnétiques sont utilisées dans de nombreux domaines, mettant à profit de leurs propriétés tout à fait avantageuses, tels que les domaines de la microélectronique, de la nanoélectronique, des aimants, mais aussi de la biomédecine, de la catalyse chimique, etc.

Les nanoparticules ferromagnétiques ont fait l'objet de nombreuses études. Parmi elles, les nanoparticules de carbure de fer s'avèrent très attractives du fait de leurs propriétés combinées de bonne stabilité à l'air, et de forte magnétisation. Elles sont ainsi considérées comme ayant un fort potentiel notamment pour la conversion et le stockage d'énergie, les nano-aimants et la nano-médecine. Parmi leurs nombreuses applications, on peut plus précisément citer l'hyperthermie magnétique et la catalyse de réactions chimiques, qui tirent profit de la capacité des nanoparticules ferromagnétiques, soumises à un champ magnétique, de convertir l'énergie externe en chaleur. La puissance générée par les nanoparticules magnétiques est gouvernée par leur débit d'absorption spécifique (SAR, pour l'anglais Specific Absorption rate).

A titre de réactions susceptibles d'être catalysées par des nanoparticules ferromagnétiques, qu'il s'agisse de nanoparticules à base de fer, de cobalt ou de nickel, on peut notamment citer les réactions de Sabatier et de Fischer-Tropsch, répondant aux schémas réactionnels suivants, classiques en eux-mêmes :

Réaction de Sabatier :

$$CO_2 + 4H_2 \rightarrow CH_4 + 2H_2O$$

Réaction de Fischer-Tropsch :

$$2(n+1)H_2 + nCO \rightarrow C_nH_{2n+2} + nH_2O$$

Les réactions de Sabatier et de Fischer-Tropsch peuvent être mises en œuvre pour le stockage de l'énergie, par réduction catalytique des oxydes de carbone en hydrocarbures : en présence d'hydrogène, par exemple produit à partir de l'énergie photovoltaïque ou éolienne, et d'un catalyseur comprenant un métal ferromagnétique tel que le fer, le cobalt, le nickel, ou leurs alliages, ou d'un catalyseur comprenant un métal noble tel que le ruthénium, le rhodium ou leurs alliages, le dioxyde de carbone est transformé en méthane (réaction de Sabatier) et le monoxyde de carbone est transformé en hydrocarbures supérieurs (procédé de Fischer-Tropsch). La réaction de Fischer-Tropsch est en particulier considérée comme l'approche la plus pratique pour produire des carburants liquides à partir des ressources fossiles telles que le gaz naturel et le charbon, ainsi que le biogaz issu de biomasse.

Il a ainsi été proposé par l'art antérieur d'utiliser des nanoparticules ferromagnétiques pour catalyser de telles réactions, en tirant profit de la capacité de ces nanoparticules à produire de la chaleur lorsqu'elles sont activées par induction magnétique. La nanoparticule catalytique activée est chauffée par le retournement de son propre moment magnétique, et sa température monte rapidement, de sorte que la réaction de catalyse démarre à sa surface, sans que le milieu réactionnel dans son ensemble n'ait atteint la température critique de réaction. On atteint ainsi de très hautes températures locales, permettant la catalyse de la réaction chimique, et ce à un faible coût énergétique.

L'emploi de nanoparticules ferromagnétiques pour la catalyse de réactions chimiques, notamment de réactions de conversion du dihydrogène et du monoxyde ou dioxyde de carbone en une autre forme chimique, et, de ce fait, la transformation de l'énergie électrique produite localement en des composés énergétiques, tels que des hydrocarbures, directement utilisables dans des systèmes thermiques, a par exemple été décrit dans le document de brevet WO-A-2014/162099.

A l'effet d'une telle catalyse, il a été proposé par l'art antérieur différents types de nanoparticules de carbure de fer.

Il a notamment été proposé, par exemple dans la publication de Yang et al., 2012, J. Am. Chem. Soc., 134, 15814-15821 des nanoparticules de carbure de fer composées de la phase cristalline $Fe_5C_2$, ou encore dans la publication de Meffre et al., 2012, Nano Letters 12, 4722-4728, des nanoparticules de carbure de fer sous forme d'un mélange de phases amorphes et cristallines incluant du $Fe_{2.2}C$ et du $Fe_5C_2$, et obtenues à partir de carbonyle de fer $Fe(CO)_5$, en vue de la catalyse de la réaction de Fischer-Tropsch.

Des nanoparticules de type à structure cœur-coquille $Fe_3C$-C ont également été proposées par l'art antérieur, comme illustré notamment dans la publication de Liu et al., 2015, Nanotechnology 26, 085601.

A l'origine de la présente invention, il a été découvert par les présents inventeurs que des nanoparticules de carbure de fer présentant une structure particulière, et notamment une phase cristalline constituée uniquement de $Fe_{2,2}C$, et une teneur particulière en $Fe_{2,2}C$, présentent, de manière tout à fait inattendue, un pouvoir de chauffe particulièrement élevé, bien plus que celui des nanoparticules de carbure de fer de l'art antérieur, et y compris lorsqu'elles sont activées par des champs magnétiques faibles. De manière plus inattendue encore, ces nanoparticules sont capables, lorsqu'elles sont activées par induction magnétique, de catalyser à elles seules les réactions de Sabatier et de Fischer-Tropsch, pour la production d'hydrocarbures, notamment de méthane, à partir de dihydrogène, et ce sans avoir recours à un quelconque autre catalyseur.

La présente invention vise ainsi à proposer des nanoparticules de carbure de fer présentant, lorsqu'elles sont activées par induction magnétique, un pouvoir de chauffe élevé, notamment amélioré par rapport aux nanoparticules ferromagnétiques proposées par l'art antérieur.

Un objectif supplémentaire de l'invention est que ce pouvoir de chauffe puisse s'exercer sous champ magnétique de faible amplitude, de sorte à réaliser des économies d'énergie.

L'invention vise également à ce que ces nanoparticules puissent être préparées par un procédé facile et rapide à mettre en œuvre, et permettant en outre un contrôle précis de la quantité de carbone présente dans le cœur de fer de la nanoparticule. Un autre objectif de l'invention est que ce procédé de préparation ne mette pas en œuvre de composés halogénés dangereux et difficiles à manipuler.

A cet effet, selon un premier aspect, il est proposé par la présente invention une nanoparticule de carbure de fer, du type à phase homogène ou à structure cœur-coquille, et comprenant une structure cristalline de $Fe_{2,2}C$, dans laquelle au moins 70 %, de préférence au moins 75 %, et préférentiellement au moins 80 %, en nombre, des atomes de fer qu'elle comporte sont présents dans ladite structure cristalline de $Fe_{2,2}C$.

Exprimé en d'autre termes, la nanoparticule selon l'invention comprend au moins 70 %, de préférence au moins 75 %, et préférentiellement au moins 80 %, en moles, par rapport au nombre total de moles de fer dans la nanoparticule, de fer participant à la phase cristalline $Fe_{2,2}C$.

La teneur de la nanoparticule en atomes de fer engagés dans la structure cristalline de $Fe_{2,2}C$ peut être déterminée par toute méthode classique en elle-même pour l'homme du métier, par exemple par spectroscopie Mössbauer, qui permet de comptabiliser les nombres relatifs d'atomes de fer impliqués dans chacune des phases composant la nanoparticule.

La nanoparticule selon l'invention peut être de phase homogène, c'est-à-dire être constituée uniquement d'une structure cristalline, ou comprendre une structure cristalline de $Fe_{2,2}C$ portant une couche non-stœchiométrique / amorphe en surface.

La nanoparticule selon l'invention peut autrement être du type à structure cœur-coquille, comportant un cœur cristallin formé essentiellement de la structure cristalline de $Fe_{2,2}C$, ce cœur pouvant en outre comprendre une quantité très minoritaire d'atomes de fer pur et/ou des impuretés sous forme de traces. La coquille de la nanoparticule peut quant à elle alors être aussi bien amorphe que polycristalline.

Une telle nanoparticule, activée par induction magnétique, présente avantageusement à la fois un pouvoir de chauffe particulièrement élevé, correspondant à un SAR supérieur à 1 kW/g, et pouvant même être supérieur à 3 kW/g, à 100 kHz et 47 mT, ainsi que la capacité de chauffer à des champs magnétiques relativement faibles, notamment d'amplitude aussi basse que 25 mT. Elles permettent ainsi de chauffer à de hautes températures à des champs magnétiques et des fréquences modérées, donc à un faible coût énergétique De telles performances sont nettement supérieures à celles obtenues avec les nanoparticules proposées par l'art antérieur, qu'il s'agisse de nanoparticules de fer, d'oxydes de fer ou de carbures de fer.

Les nanoparticules selon l'invention présentent en outre l'avantage de montées et descentes en température très rapides, si bien qu'il en résulte une économie d'énergie plus grande encore lors de leur mise en œuvre.

Elles présentent par ailleurs la capacité de catalyser des réactions chimiques nécessitant un apport de chaleur, ainsi qu'à catalyser à elles seules la réaction de Sabatier, de réduction du dioxyde de carbone en hydrocarbures, sans dopage par un élément autre tel que le cobalt ou le ruthénium.

Elles trouvent plus généralement application pour tout type de transformation catalytique en phase gazeuse ou liquide utilisant comme moyen de chauffage l'induction magnétique.

Les nanoparticules selon l'invention sont de préférence de type monodomaine, c'est-à-dire d'une taille inférieure à la taille critique de transition entre état monodomaine et état multidomaine.

Préférentiellement, leur taille comprise entre 1 et 20 nm, et de préférence comprise entre 10 et 16 nm.

On entend par là que chacune de leurs dimensions est comprise entre environ 1 et environ 20 nm, notamment comprise entre 10 et 16 nm.

Préférentiellement, leur taille est égale à 15 nm ± 1 nm. Une telle caractéristique confère notamment aux nanoparticules les performances les plus élevées en terme de pouvoir de chauffe.

Les nanoparticules selon l'invention peuvent présenter toute forme. La forme sensiblement sphérique est cependant particulièrement préférée dans le cadre de l'invention.

Elles présentent de préférence une bonne monodispersité. On entend par là une distribution de taille de +/- 10 % au maximum par rapport à la taille moyenne.

Les nanoparticules selon l'invention peuvent contenir en outre un composé catalyseur d'une réaction chimique donnée, tel qu'un métal catalytique, qui est présent sur au moins une partie de leur surface, de sorte à améliorer leur activité catalytique pour cette réaction chimique particulière, par une combinaison de propriétés physiques et de propriétés chimiques lui permettant de jouer le rôle à la fois de catalyseur de la réaction, et de fournisseur de l'énergie thermique nécessaire à la réaction, après stimulation par induction magnétique.

Ainsi, dans des modes de réalisation particuliers de l'invention, les nanoparticules de carbure de fer sont recouvertes, sur au moins une partie de leur surface, d'un revêtement d'un métal catalytique.

La composition d'un tel revêtement est avantageusement choisie pour permettre, en fonction de la réaction chimique particulière visée, de catalyser cette réaction, d'augmenter son rendement et/ou d'améliorer sa sélectivité.

Dans des configurations particulières des nanoparticules selon l'invention, dans lesquelles le revêtement de métal catalytique recouvre entièrement la surface des nanoparticules, le métal catalytique joue le rôle de catalyseur de la réaction chimique, le carbure de fer lui fournissant l'énergie thermique nécessaire à cet effet.

Dans d'autres configurations particulières des nanoparticules selon l'invention, dans lesquelles le revêtement de métal catalytique ne recouvre qu'une partie de la surface des nanoparticules, le carbure de fer est exposé au milieu réactionnel, et peut jouer à la fois le rôle de catalyseur de la réaction chimique et de source d'énergie thermique pour sa propre action catalytique, ainsi que pour l'action catalytique combinée du métal catalytique.

Le métal catalytique peut notamment être choisi parmi le nickel, le ruthénium, le cobalt, le cuivre, le zinc, le platine, le palladium, le rhodium, ou encore le manganèse, le molybdène, le tungstène, le vanadium, l'iridium ou l'or, ou l'un quelconque de leurs alliages, pris seuls ou en mélange, par exemple sous forme d'un mélange cuivre/zinc.

Pour la catalyse de la réaction de Sabatier et/ou de la réaction de Fischer-Tropsch, la performance des nanoparticules de carbure de fer selon l'invention peut notamment être améliorée par un dépôt de nickel formé sur leur surface.

Les nanoparticules de carbure de fer selon l'invention sont notamment susceptibles d'être obtenues par une étape de carburation de nanoparticules de fer zéro-valent, par mise en présence de ces nanoparticules de fer zéro-valent avec un mélange gazeux de dihydrogène et de monoxyde de carbone.

Dans des modes de réalisation particuliers de l'invention, la nanoparticule de carbure de fer est supportée sur un support solide.

Ce support solide se présente notamment sous forme pulvérulente.

Ce support solide est choisi pour, d'une part, être inerte vis-à-vis de la réaction pour laquelle les nanoparticules selon l'invention sont destinées à être utilisées, et, d'autre part, assurer un bon maintien en place des nanoparticules pendant cette réaction. Le support solide peut notamment être formé en un matériau choisi parmi les oxydes métalliques micro- ou mésoporeux, le carbone, ou l'un quelconque de leurs mélanges ou alliages. Il peut par exemple être formé en alumino-silicate, tel que le support Siralox® commercialisé par la société Sasol, ou encore en oxyde de zirconium, en oxyde de cérium, etc.

Le taux de chargement du support solide par les nanoparticules selon l'invention peut notamment être compris entre 1 et 50 % en poids de fer, par rapport au poids total du support.

Le support solide peut, le cas échéant, être par ailleurs dopé par un métal catalytique. Ce métal catalytique peut notamment être choisi parmi le nickel, le ruthénium, le cobalt, le cuivre, le zinc, le platine, le palladium, le rhodium, ou encore le manganèse, le molybdène, le tungstène, le vanadium, l'iridium ou l'or, ou l'un quelconque de leurs alliages, pris seuls ou en mélange, par exemple sous forme d'un mélange cuivre/zinc.

Le support solide peut aussi, le cas échéant, être dopé par un agent dopant classique en lui-même pour la réaction de catalyse chimique visée, tel qu'un agent alcalin, un agent alcalino-terreux, etc., ou un mélange de tels agents dopants.

Selon un deuxième aspect, la présente invention concerne un procédé de préparation de nanoparticules de carbure de fer selon l'invention, pouvant répondre à l'une ou plusieurs des caractéristiques ci-avant. Ce procédé comprend une étape de carburation de nanoparticules de fer zéro-valent, par mise en présence desdites nanoparticules de fer zéro-valent avec un mélange gazeux de dihydrogène et de monoxyde de carbone. La mise en œuvre d'une telle étape de carburation permet avantageusement d'obtenir des nanoparticules de carbure de fer de structure cœur-coquille dont le cœur est un cœur cristallin composé exclusivement de $Fe_{2,2}C$.

Une telle étape de carburation s'avère en outre tout à fait avantageuse en ce qu'elle permet, par un choix adéquat de ses paramètres opératoires, de contrôler avec précision la quantité de carbone introduite dans les nanoparticules de fer zéro-valent, et donc la teneur molaire en phase cristalline de $Fe_{2,2}C$ dans la nanoparticule, et d'influer ainsi sur les caractéristiques des nanoparticules qui déterminent leur capacité de chauffe.

Selon des modes de mise en œuvre particuliers de l'invention, le procédé de préparation des nanoparticules répond en outre aux caractéristiques suivantes, mises en œuvre séparément ou en chacune de leurs combinaisons

techniquement opérantes.

Dans des modes de mise en œuvre particuliers de l'invention, l'étape de carburation est réalisée à une température comprise entre 120 et 300 °C, de préférence comprise entre 120 et 180 °C, et préférentiellement d'environ 150 °C. Des températures supérieures à 300 °C induisent en particulier un changement de phase, et mènent à l'obtention d'une teneur élevée en structure cristalline de $Fe_5C_2$, ce qui est contraire à la présente invention.

L'étape de carburation est de préférence réalisée pendant une durée comprise entre 72 et 200 h. Dans cette fourchette, le choix de la durée exacte de l'étape de carburation permet de contrôler la teneur en carbone des nanoparticules, et de ce fait leurs propriétés hyperthermiques.

Dans des modes de mise en œuvre particuliers de l'invention, l'étape de carburation comprend l'élimination de l'eau formée lors de la réaction des nanoparticules de fer zéro-valent et du mélange gazeux, au fur et à mesure de sa formation.

Cette élimination peut notamment être réalisée au moyen d'un tamis moléculaire, de taille de pores adaptée au piégeage des molécules d'eau, placé dans une zone du réacteur choisie de sorte à ce qu'il ne soit pas en contact avec les nanoparticules, et pas exposé à de hautes températures.

L'élimination de l'eau in-situ au fur et à mesure de sa formation permet avantageusement d'accélérer la réaction de carburation des nanoparticules de fer zéro-valent, et d'obtenir des nanoparticules de carbure de fer conformes à la présente invention de manière bien plus rapide qu'en l'absence d'élimination de l'eau formée lors de la réaction. Ainsi, en des durées de carburation comprises entre 24 et 60 h par exemple, on obtient des nanoparticules aux performances de chauffe particulièrement élevées.

De manière générale, il est du ressort de l'homme du métier de déterminer, pour chacun des paramètres opératoires ci-avant et ci-après, la valeur exacte à appliquer, notamment à l'intérieur des fourchettes préférentielles indiquées dans la présente description, de sorte à obtenir les propriétés particulières souhaitées pour les nanoparticules de carbure de fer, en fonction de l'application particulière visée.

L'étape de carburation peut être réalisée par mise en contact du mélange gazeux avec des nanoparticules soit sous forme d'une dispersion dans un solvant, de préférence un solvant organique aprotique, tel que le mésitylène, soit sous forme de poudre.

Elle peut par exemple mettre en œuvre une pression de dihydrogène comprise entre 1 et 10 bars, préférentiellement d'environ 2 bars, et/ou une pression de monoxyde de carbone comprise entre 1 et 10 bars, préférentiellement d'environ 2 bars.

Dans des modes de mise en œuvre particuliers de l'invention, le procédé comporte une étape préalable de préparation des nanoparticules de fer zéro-valent ($Fe^0$) par décomposition d'un précurseur organométallique répondant à la formule générale (I) :

$$Fe(NR^1R^2)(NR^3R^4) \qquad (I)$$

dans laquelle $R^1$, $R^2$, $R^3$ et $R^4$, identiques ou différents, représentent chacun un groupement alkyle, aryle, triméthylsilyle ou triméthylalkyle,

en présence de dihydrogène et d'un système de ligands comprenant un acide carboxylique et une amine, de préférence une amine primaire ou une amine secondaire, au moins un composé parmi cet acide carboxylique et cette amine comportant une chaine hydrocarbonée en C4 à C34, de préférence en C8 à C20.

Sont notamment exclus, dans le cadre de la présente invention, les précurseurs de formule générale $Fe(COT)_2$ ou $Fe(CO)_5$, ou tout dérivé carbonyle de fer, tel que $Fe_3(CO)_{12}$,et tout ferrocène Fe(Cp)2 ou tout dérivé du ferrocène.

Un précurseur organométallique particulièrement préféré est le dimère de bis(triméthylsilyl)amido-fer(II).

L'étape de préparation des nanoparticules de fer zéro-valent peut notamment être mise en œuvre sous une pression de dihydrogène comprise entre 1 et 10 bars, préférentiellement environ égale à 2 bars.

L'acide carboxylique et l'amine contenus dans le système de ligands peuvent, l'un comme l'autre, être aussi bien linéaires que ramifiés ou cycliques. Ils peuvent être fonctionnalisés ou non fonctionnalisés, à chaîne saturée aussi bien qu'insaturée.

Dans des modes de mise en œuvre particulièrement préférés de l'invention, le système de ligands comprend l'acide palmitique et/ou l'hexadécylamine, de préférence le couple acide palmitique / hexadécylamine.

Le procédé selon l'invention permet alors avantageusement de préparer, en composés intermédiaires, des nanoparticules de $Fe^0$ de forme sensiblement sphérique et surtout monodisperses, permettant de préparer des nanoparticules de carbure de fer également sensiblement sphériques et monodisperses. Il en résulte une grande homogénéité de chauffage par les nanoparticules de carbure selon l'invention.

Par ailleurs, l'utilisation d'un tel système de ligands acide palmitique / hexadécylamine permet avantageusement d'obtenir des pourcentages molaires de la structure cristalline de $Fe_{2,2}C$ dans la nanoparticule supérieurs ou égaux à 70 %, conformément à ce qui est préconisé par la présente invention.

Elle permet en outre de réaliser une carburation directe des nanoparticules de $Fe^0$ obtenues, c'est-à-dire par

mise en contact de ces nanoparticules directement avec la phase gazeuse.

Ainsi, dans des modes de mise en œuvre particuliers de l'invention, l'étape de carburation est mise en œuvre directement sur les nanoparticules de fer zéro-valent obtenues à l'issue de l'étape de décomposition du procédé.

Le procédé selon l'invention peut en outre répondre à l'une ou plusieurs, de préférence l'ensemble, des caractéristiques ci-après, concernant l'étape de décomposition du précurseur organométallique, pour former les nanoparticules de $Fe^0$ :

- l'étape de décomposition est mise en œuvre à une température comprise entre 120 et 300 °C, de préférence comprise entre 120 et 180 °C, et préférentiellement à environ 150 °C ;
- l'étape de décomposition est mise en œuvre pendant une durée comprise entre 1 et 72 h, de préférence d'environ 48 h ;
- l'étape de décomposition est mise en œuvre dans un solvant organique aprotique à point d'ébullition supérieur à 100 °C, notamment un solvant aromatique, par exemple le toluène ou le mésitylène.

De telles caractéristiques permettent avantageusement d'améliorer la maîtrise des propriétés des nanoparticules formées.

Le procédé selon l'invention, répondant à l'une ou plusieurs des caractéristiques ci-avant, est avantageusement simple à mettre en œuvre. Il s'avère en outre plus avantageux en de nombreux points que les procédés de préparation de nanoparticules de carbure de fer proposées par l'art antérieur, outre le fait qu'il permet de préparer des nanoparticules à teneur élevée en structure cristalline de $Fe_{2,2}C$, et qu'il permet de contrôler précisément la quantité de carbone introduite dans cette structure cristalline, permettant ainsi de maîtriser la teneur en $Fe_{2,2}C$ dans la nanoparticule.

Par rapport aux procédés de l'art antérieur mettant en œuvre un système de ligands hexadécylamine / chlorure d'hexadécylammonium, le mode de mise en œuvre particulièrement préféré de l'invention utilisant le système acide palmitique / hexadécylamine présente notamment l'avantage d'éviter les risques de modification des propriétés magnétiques et catalytiques des nanoparticules provoquée par l'action du chlore.

Par rapport aux procédés de l'art antérieur mettant en œuvre du pentacarbonyle de fer $Fe(CO)_5$ pour réaliser la carburation de nanoparticules de fer, le procédé selon l'invention s'avère moins dangereux et moins difficile à réaliser, et il permet un contrôle bien plus précis de la carburation ainsi que l'obtention d'un cœur de la phase cristalline pure $Fe_{2,2}C$.

Lorsqu'il est souhaité de préparer des nanoparticules de carbure de fer recouvertes en surface, au moins partiellement, d'un métal catalytique, le procédé selon l'invention peut comprendre une étape ultérieure de traitement des nanoparticules de carbure de fer selon l'invention par mise en contact avec un précurseur organométallique dudit métal catalytique, par exemple un précurseur de nickel, cette mise en contact pouvant notamment, mais non obligatoirement, être réalisée en présence d'hydrogène.

Une telle étape de traitement de nanoparticules par la voie dite organométallique est classique en elle-même, et peut être réalisée de toute manière connue de l'homme du métier.

Un autre aspect de l'invention concerne l'utilisation de nanoparticules de carbure de fer selon l'invention, pouvant répondre à l'une ou plusieurs des caractéristiques ci-avant, pour la production de chaleur, après activation par induction magnétique. Les nanoparticules de carbure de fer peuvent notamment être utilisées pour l'hyperthermie, dans le domaine de la biomédecine, selon des protocoles d'utilisation classiques en eux-mêmes, et tirant parti de leur SAR particulièrement élevé.

La présente invention concerne en outre l'utilisation de nanoparticules de carbure de fer selon l'invention, pouvant répondre à l'une ou plusieurs des caractéristiques ci-avant, pour la catalyse de réactions chimiques, toujours par activation par induction magnétique.

La réaction chimique peut notamment être une réaction de réduction de dioxyde de carbone ou de monoxyde de carbone en hydrocarbure(s), telle qu'une réaction de Sabatier ou de Fischer-Tropsch, réaction que les nanoparticules de carbure de fer selon l'invention sont avantageusement aptes à catalyser à elles seules, sans adjonction d'un catalyseur spécifique supplémentaire.

Ainsi, les nanoparticules selon l'invention peuvent avantageusement être utilisées pour le stockage chimique d'énergie sous forme d'hydrocarbure(s), par exemple sous forme de méthane.

Pour toutes ces applications, les nanoparticules sont soumises à un champ magnétique d'amplitude préférentiellement comprise entre 10 et 65 mT, de fréquence comprise entre 100 et 300 kHz. Les moyens de génération de ce champ magnétique sont classiques en eux-mêmes.

Selon un aspect supplémentaire, la présente invention porte ainsi sur un procédé de catalyse d'une réaction chimique au moyen de nanoparticules de carbure de fer selon la présente invention, pouvant répondre à l'une ou plusieurs des caractéristiques ci-avant. Selon ce procédé, les nanoparticules de carbure de fer sont introduites dans un milieu réactionnel contenant un ou des réactifs de la réaction chimique visée, et le milieu réactionnel est soumis à un

champ magnétique apte à provoquer une augmentation de la température des nanoparticules jusqu'à une température supérieure ou égale à une température nécessaire à la réalisation de la réaction chimique.

L'activation des nanoparticules par induction magnétique est de préférence réalisée à partir d'un inducteur de champ externe au réacteur dans lequel la réaction est mise en œuvre. On entend par inducteur, tout système d'induction magnétique comprenant des organes générant un champ magnétique, des organes permettant de contrôler les valeurs de ce champ magnétique, ainsi que son alimentation, qui peut être électrique ou autre. En particulier, les organes générant le champ magnétique peuvent être placés dans le réacteur, dans sa paroi, ou à l'extérieur du réacteur.

Dans des modes de mise en œuvre particuliers de l'invention, le champ magnétique est appliqué à une première amplitude, de préférence supérieure à 50 mT, pendant une première période de temps, de préférence d'une durée comprise entre 3 secondes et 1 minute, puis à une deuxième amplitude, inférieure à ladite première amplitude, de préférence comprise entre 20 et 40 mT, pendant une deuxième période de temps, ladite deuxième période de temps étant plus longue que ladite première période de temps, et étant de préférence supérieure ou égale à 4 heures. Un tel mode de mise en œ uvre s'avère notamment tout à fait avantageux du point de vue de la faible consommation d'énergie nécessaire à la réalisation de la réaction chimique.

Dans des modes de mise en œuvre particuliers de l'invention, notamment particulièrement adaptés aux configurations dans lesquelles les nanoparticules de carbure de fer sont recouvertes d'un revêtement d'un métal catalytique, par exemple de nickel, le champ magnétique est appliqué au milieu réactionnel de manière puisée.

Dans des modes de mise en œuvre particuliers de l'invention, les nanoparticules sont supportées sur un support solide, et la réaction chimique est réalisée en flux de réactif(s) continu(s). Ainsi, les nanoparticules selon l'invention sont placées dans un réacteur, et le ou les réactifs de la réaction sont entrainés à travers ce réacteur en flux continu. La capacité de chauffe des nanoparticules selon l'invention est alors avantageusement suffisamment élevée pour permettre l'obtention d'une température adaptée à la catalyse de la réaction chimique visée, malgré les faibles temps de contact se produisant entre les nanoparticules et le ou les réactifs.

Le procédé selon l'invention peut aussi bien être utilisé pour la catalyse en phase gazeuse, dans laquelle les réactifs se trouvent sous forme gazeuse, que pour la catalyse en phase liquide, dans laquelle les réactifs se trouvent sous forme liquide.

En fonction du métal catalytique présent à leur surface, les nanoparticules de carbure de fer selon l'invention peuvent en outre être utilisées pour de nombreuses autres applications, par exemple, non limitativement :

- pour la synthèse du méthanol, revêtues en surface d'un mélange de cuivre et de zinc Cu/Zn,
- pour la catalyse de réactions d'hydrogénation, ou en tant que matériaux d'électrodes, revêtues en surface de palladium ou de platine,
- pour la catalyse de réactions de carbonylation ou d'hydrogénation, revêtues en surface de rhodium,
- pour la catalyse de réactions de Sabatier ou de Fischer-Tropsch, avec une sélectivité améliorée, comme exposé ci-avant, recouvertes de cobalt, de nickel ou de ruthénium.

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 25, dans lesquelles :

- la figure 1 montre les résultats de tests de caractérisation de nanoparticules de $Fe^0$ de 9,0 nm préparées conformément à l'invention, (a) par microscopie électronique en transmission, (b) par diffraction des rayons X ;
- la figure 2 montre les résultats de tests de caractérisation de nanoparticules de $Fe^0$ de 12,5 nm préparées conformément à l'invention, (a) par microscopie électronique en transmission, (b) par diffraction des rayons X ;
- la figure 3 montre les résultats de tests de caractérisation de nanoparticules de carbure de fer de 13,0 nm à 80 % molaire de $Fe_{2,2}C$ conformes à l'invention, (a) et (b) par microscopie électronique en transmission avec deux grossissements différents, (c) par diffraction des rayons X, (d) par spectroscopie Môssbauer ;
- la figure 4 montre les résultats de tests de caractérisation de nanoparticules de carbure de fer de 15,0 nm à 80 % molaire de $Fe_{2,2}C$ conformes à l'invention, (a) par microscopie électronique en transmission, (b) par diffraction des rayons X, (c) par spectroscopie Môssbauer ;
- la figure 5 montre les résultats de tests de caractérisation de nanoparticules de carbure de fer de 9,7 nm à 59 %

molaire de $Fe_{2,2}C$ conformes à l'invention, (a) par microscopie électronique en transmission, (b) par diffraction des rayons X, (c) par spectroscopie Môssbauer ;

- la figure 6 montre les résultats de tests de caractérisation de nanoparticules de carbure de fer à 80 % molaire de $Fe_{2,2}C$ recouvertes de nickel conformes à l'invention, (a) par microscopie électronique en transmission, (b) par diffraction des rayons X, (c1) par STEM, (c2) par STEM-EDX ciblée sur le fer et (c3) par STEM-EDX ciblée sur le nickel ;

- la figure 7 représente un graphe montrant le débit spécifique d'absorption (SAR), en fonction de l'amplitude du champ magnétique, pour un test d'hyperthermie par induction magnétique à 100 kHz réalisé sur des nanoparticules d'oxyde de fer de l'art antérieur (FeONP), des nanoparticules de fer préparées selon un procédé conforme à l'invention (FeNP2), des nanoparticules de carbure de fer conformes à l'invention (FeCNP2) et des nanoparticules de carbure de fer selon l'art antérieur (FeCcomp1) ;

- la figure 8 représente un graphe montrant le débit spécifique d'absorption (SAR), en fonction de l'amplitude du champ magnétique, pour un test d'hyperthermie par induction magnétique à 100 kHz réalisé sur des nanoparticules de fer préparées selon un procédé conforme à l'invention (FeNP1), des nanoparticules de carbure de fer conformes à l'invention (FeCNP1, FeCNP3, FeCNP4 et FeCNP5) et des nanoparticules de carbure de fer comparatives (FeCcomp2, FeCcomp3, FeCcomp4, FeCcomp6, FeCcomp7) ;

- la figure 9 représente un graphe montrant le débit spécifique d'absorption (SAR), en fonction de l'amplitude du champ magnétique, pour un test d'hyperthermie par induction magnétique à 100 kHz réalisé sur des nanoparticules de fer préparées selon un procédé conforme à l'invention (FeNP2), des nanoparticules de carbure de fer conformes à l'invention (FeCNP2) et des nanoparticules de carbure de fer comparatives (FeCcomp8, FeCcomp9, FeCcomp10) ;

- la figure 10 représente un graphe montrant le débit spécifique d'absorption (SAR), en fonction de l'amplitude du champ magnétique, pour un test d'hyperthermie par induction magnétique à 100 kHz réalisé sur des nanoparticules de carbure de fer conformes à l'invention de différentes tailles FeCNP1 et FeCNP2 ;

- la figure 11 représente un graphe montrant le débit spécifique d'absorption (SAR), en fonction de l'amplitude du champ magnétique, pour un test d'hyperthermie par induction magnétique à 100 kHz réalisé sur des nanoparticules de carbure de fer conformes à l'invention (FeCNP2) et des nanoparticules de carbure de fer recouvertes de nickel conformes à l'invention (FeC@Ni) ;

- la figure 12 représente un graphe montrant d'une part le taux de conversion du $CO_2$, et d'autre part le rendement en hydrocarbures, en fonction de l'amplitude du champ magnétique appliqué, lors de la mise en œuvre de nanoparticules de carbure de fer conformes à l'invention pour la catalyse de la réaction de Sabatier ;

- la figure 13 montre le spectre de masse de la phase gazeuse obtenue à l'issue de la mise en œuvre de nanoparticules de carbure de fer conformes à l'invention pour la catalyse de la réaction de Sabatier, sous champ magnétique de 30 mT à 300 kHz ;

- la figure 14 montre le spectre de masse de la phase gazeuse obtenue à l'issue de la mise en œuvre de nanoparticules de carbure de fer conformes à l'invention pour la catalyse de la réaction de Sabatier, sous champ magnétique de 40,2 mT à 300 kHz ;

- la figure 15 montre le diffractogramme des rayons X de nanoparticules de carbure de fer conformes à l'invention à l'issue d'une réaction de catalyse de la réaction de Sabatier, sous champ magnétique de 40,2 mT à 300 kHz pendant 8 h ;

- la figure 16 montre les spectres Môssbauer obtenus pour des nanoparticules selon l'invention (b/ obtenues avec un temps de carburation de 96 h, c/ obtenues avec un temps de carburation de 140 h) et pour des nanoparticules de carbure de fer préparées selon le même protocole, mais avec un temps de carburation de 48 h (a/), non conformes à l'invention ;

- la figure 17 représente un graphe montrant le débit spécifique d'absorption (SAR), en fonction de l'amplitude du champ magnétique, pour un test d'hyperthermie par induction magnétique à 100 kHz réalisé sur des nanoparticules de fer zéro-valent ($Fe^0$), et des nanoparticules de carbure de fer préparées à partir de ces nanoparticules de fer

zéro-valent : selon un procédé conforme à l'invention mettant en œuvre un temps de carburation de 96 h (NP96) ou un temps de carburation de 140 h (NP140), ou selon un procédé mettant en œuvre un temps de carburation de 48 h (NP48) ;

- la figure 18 représente un graphe montrant le débit spécifique d'absorption (SAR), en fonction de l'amplitude du champ magnétique, pour un test d'hyperthermie par induction magnétique à 100 kHz réalisé sur des nanoparticules de carbure de fer préparées : selon un procédé conforme à l'invention mettant en œuvre pour l'étape de carburation un tamis moléculaire et un temps de carburation de 40 h (NP40TM) ou pas de tamis moléculaire et un temps de carburation de 140 h (NP140S), ou selon un procédé mettant en œ uvre un temps de carburation 48 h sans tamis moléculaire (NP48S) ;

- la figure 19 représente un graphe montrant le débit spécifique d'absorption (SAR), en fonction de l'amplitude du champ magnétique, pour un test d'hyperthermie par induction magnétique à 100 kHz réalisé sur des nanoparticules de carbure de fer préparées : selon un procédé conforme à l'invention mettant en œuvre pour l'étape de carburation un tamis moléculaire et un temps de carburation de 24 h (NP24TM, expérience réalisée en double) ou pas de tamis moléculaire et un temps de carburation de 120 h (NP120S), ou selon un procédé mettant en œuvre un temps de carburation 24 h sans tamis moléculaire (NP24S) ;

- la figure 20 montre un graphe représentant, en fonction de l'amplitude du champ magnétique appliqué, le taux de conversion du dioxyde de carbone ($CO_2$) et les taux de formation de méthane ($CH_4$) et de monoxyde de carbone (CO) lors de la mise en œuvre d'un procédé de catalyse de la réaction de Sabatier selon l'invention, réalisé en flux de réactifs continus, mettant en œuvre des nanoparticules de carbure de fer recouvertes de nickel et supportées sur support solide conformes à l'invention ;

- la figure 21 représente un chromatogramme en phase gazeuse obtenu en sortie du réacteur lors de la mise en œuvre du procédé de la figure 20, pour une amplitude de champ magnétique de 40 mT ;

- la figure 22 montre une image de microscopie électronique, d'un grain de support solide dopé au ruthénium et supportant des nanoparticules de carbure de fer conformes à l'invention ;

- la figure 23 montre un graphe représentant, en fonction de l'amplitude du champ magnétique appliqué, le taux de conversion du dioxyde de carbone ($CO_2$), le taux de formation de monoxyde de carbone (CO) et la sélectivité de formation du méthane ($CH_4$) lors de la mise en œuvre d'un procédé de catalyse de la réaction de Sabatier selon l'invention, réalisé en flux de réactifs continus, mettant en œuvre des nanoparticules de carbure de fer recouvertes et supportées sur support solide dopé au ruthénium conformes à l'invention ;

- la figure 24 représente un chromatogramme en phase gazeuse obtenu en sortie du réacteur lors de la mise en œuvre du procédé de la figure 23, pour une amplitude de champ magnétique de 28 mT ;

- et la figure 25 représente un graphe montrant, en fonction du temps, l'évolution de la température dans le réacteur, le taux de conversion du dioxyde de carbone ($CO_2$), le taux de formation de monoxyde de carbone (CO) et la sélectivité de formation du méthane ($CH_4$) lors de la mise en œuvre du procédé de la figure 23, à un champ magnétique d'amplitude 28 mT.

## A/ Matériel et méthodes

Toutes les synthèses de composés non commerciaux ont été effectuées sous argon en utilisant des bouteilles Fischer-Porter, une boîte à gant et une rampe à vide/argon. Le mésitylène (99%), toluène (99%) et le tétrahydrofurane (THF, 99%) ont été achetés chez VWR Prolabo, purifiés sur alumine et dégazés par trois cycles congélation-pompage-liquéfaction. Les produits commerciaux hexadécylamine (HDA, 99%) et acide palmitique (AP) ont été achetés chez Sigma-Aldrich. Le dimère bis(amido)iron(II) $\{Fe[N(SiMe_3)_2]_2\}_2$ et le (1,5-cyclooctadiene)(1,3,5-cyclooctatriene)ruthenium(0) (Ru(COD)(COT)) ont été achetés chez NanoMePS. Le nickel(II)acetylacetonate a été acheté chez Sigma Aldrich. Les nanoparticules de Fe(0) de 9,0 nm ont été soit synthétisées, soit achetées chez NanoMePS. Tous ces composés ont été utilisés sans purification supplémentaire.

Le tamis moléculaire (pores 0,4 nm, diamètre 2 mm) a été acheté chez Merck (CAS 1318-02-1) et a été activé sous vide à 200 °C pendant 3 h. Le support Siralox® a été obtenu auprès de la société Sasol.

Caractérisation

La taille et la morphologie des échantillons synthétisés ont été caractérisées par microscopie électronique à transmission (MET). Les images de microscopie conventionnelles ont été obtenues en utilisant un microscope JEOL (model 1400) fonctionnant à 120 kV. Les mesures de diffraction des rayons X (DRX) ont été effectuées sur un diffractomètre PANalyticalEmpyrean en utilisant une source Co-Ka à 45 kV et 40 mA. Ces études ont été conduites sur des échantillons en poudre préparés et scellés sous argon. Les analyses de spectrométrie de masse ont été réalisées sur un spectromètre Pfeiffer VacuumThermostar™GasAnalysis System GSD 320. L'état des atomes de fer et leur environnement a été déterminé par spectroscopie Môssbauer (Wissel, 57Co source).

Les analyses de chromatographie en phase gazeuse couplée à la spectrométrie de masse (GC-MS) ont été effectuées sur un chromatographe en phase gazeuse PerkinElmer 580 couplé à un spectromètre de masse Clarus® SQ8T. La conversion du dioxyde de carbone $CO_2$, le rendement en méthane $CH_4$, le rendement en monoxyde de carbone CO et la sélectivité en méthane ont été calculés à partir des chromatogrammes en phase gazeuse, après calibration du détecteur TCD. Les mesures magnétiques ont été effectuées sur un magnétomètre à échantillon vibrant (Vibrating Sample Magnetometer, Quantum Device PPMS EverCool-II®). Les nanoparticules ont été préalablement diluées (100 fois) dans le tétracosane afin de supprimer les interactions magnétiques.

**B/ Synthèse de nanoparticules de fer zéro-valent**

Protocole général

En boîte à gants, le précurseur de fer $\{Fe[N(SiMe_3)_2]_2\}_2$, l'acide palmitique et l'hexadécylamine sont pesés séparément dans des piluliers de 15 mL et solubilisés dans le mésitylène. La solution verte contenant le précurseur de fer est introduite dans une bouteille Fischer-Porter, suivie par l'acide palmitique et l'hexadécylamine. La bouteille Fischer-Porter est sortie de la boîte à gants et placée sous agitation dans un bain d'huile à 32 °C. Elle est ensuite purgée de son argon et mise sous pression de dihydrogène, entre 1 et 10 bars. Le mélange est agité vigoureusement à une température comprise entre 120 et 180 °C pendant 1 à 72h.

Une fois la réaction terminée, la bouteille Fischer-Porter est retirée du bain d'huile et laissée à refroidir sous agitation. Une fois à température ambiante, elle est entrée en boîte à gants et dégazée. Les nanoparticules de fer obtenues sont lavées par décantation magnétique, trois fois au toluène et trois fois au THF. Pour finir, les nanoparticules de fer sont séchées sous rampe à vide. Elles sont ensuite caractérisées par microscopie électronique en transmission (TEM), diffraction des rayons X (DRX), magnétomètrie à échantillon vibrant (VSM) et analyse élémentaire (ATG).

Exemple 1 - synthèse de nanoparticules de $Fe^0$ de 9,0 nm

Le protocole général ci-dessus est appliqué avec les paramètres suivants : le précurseur de fer $\{Fe[N(SiMe_3)_2]_2\}_2$ (1,0 mmol ; 753,2 mg), l'acide palmitique (1,2 équivalent/Fe, 2,4 mmol; 615,4 mg) et l'hexadécylamine (1 équivalent/Fe, 2,0 mmol ; 483,0 mg) sont solubilisés respectivement dans 5 mL, 10 mL et 5 mL de mésitylène.

La solution verte contenant le précurseur de fer est introduite dans une bouteille Fischer-Porter (rinçage du pilulier avec 5 mL de mésitylène), suivie par l'acide palmitique (rinçage du pilulier avec 10 mL de mésitylène) et l'hexadécylamine (rinçage du pilulier avec 5 mL de mésitylène). La bouteille de Fischer-Porter est fermée, sortie de la boite à gants et placée sous agitation dans un bain d'huile à 32°C. Elle est ensuite purgée de son argon et mise sous pression d'hydrogène (2 bars). Le mélange est agité vigoureusement à 150 °C pendant 48 h.

Les nanoparticules obtenues, nommées ci-après FeNP1, sont caractérisées. Les résultats obtenus par TEM et DRX sont montrés sur la figure 1, respectivement en (a) et (b). On observe qu'elles sont sphériques, monodisperses, de diamètre D = 9,0 nm +/- 0,5 nm, et formées d'une phase cristalline de $Fe^0$ bcc.

Exemple 2 - synthèse de nanoparticules de $Fe^0$ de 12,5 nm

Le protocole général ci-dessus est appliqué avec les paramètres suivants : le précurseur de fer $\{Fe[N(SiMe_3)_2]_2\}_2$ (1,0 mmol ; 753,2 mg), l'acide palmitique (1,3 équivalent/Fe, 2,6 mmol ; 666,4 mg) et l'hexadécylamine (1 équivalent/Fe, 2,0 mmol ; 483,0 mg) sont solubilisés respectivement dans 5 mL, 10 mL et 5 mL de mésitylène. Les étapes suivantes sont réalisées conformément à l'Exemple 1 ci-avant. Le reste de la synthèse ainsi que la purification et la caractérisation sont poursuivies comme dans l'exemple 1.

Les nanoparticules obtenues, nommées ci-après FeNP2, sont caractérisées. Les résultats obtenus par TEM et DRX sont montrés sur la figure 2, respectivement en (a) et (b). On observe qu'elles sont sphériques, monodisperses, de diamètre D = 12,5 nm +/- 0,7 nm, et formées d'une phase cristalline de $Fe^0$bcc.

**C/ Synthèse de nanoparticules de carbure de fer**

Protocole général

En boîte à gant, les nanoparticules de $Fe^0$ sont placées dans une bouteille Fischer-Porter et redispersées dans le mésitylène. La bouteille Fischer-Porter est fermée et sortie de la boîte à gant, purgée de son argon puis mise sous pression de monoxyde de carbone (entre 1 et 10 bars) et d'hydrogène (entre 1 et 10 bars). Le mélange est ensuite agité vigoureusement à 120-180 °C pendant une durée comprise entre 1 min et 200 h.

Une fois la réaction terminée, la bouteille Fischer-Porter est retirée du bain d'huile et laissée à refroidir sous agitation. Une fois à température ambiante, elle est entrée en boite à gant et dégazée. Les nanoparticules obtenues sont lavées, par lavage magnétique, 3 fois au toluène, puis séchées sous rampe à vide. La poudre noire obtenue est analysée en TEM, DRX, VSM, spectroscopie Môssbauer et analyse élémentaire.

Exemple 3 - synthèse de nanoparticules de carbure de fer de 13,0 nm contenant 83 % de la structure cristalline $Fe_{2.2}C$

Le protocole général ci-dessus est appliqué avec les paramètres suivants : les nanoparticules de $Fe^0$ obtenues à l'Exemple 2 ci-avant (1 mmol Fe ; 100 mg) sont placées dans une bouteille Fischer-Porter et redispersées dans le mésitylène (20 mL). La bouteille Fischer-Porter est mise sous pression de monoxyde de carbone (2 bars) et d'hydrogène (2 bars). Le mélange est ensuite agité vigoureusement à 150 °C pendant 120 h.

Les nanoparticules obtenues, nommées ci-après FeCNP1, sont caractérisées. Les résultats obtenus par TEM (à deux grossissements différents), DRX et spectroscopie Môssbauer sont montrés sur la figure 3, respectivement en (a), (b), (c) et (d). On observe que les nanoparticules sont sphériques, monodisperses, de diamètre D = 13,1 nm +/- 1,1 nm, et qu'elles comprennent un cœur monocristallin de $Fe_{2,2}C$. Il ressort de la spectroscopie Môssbauer que leur teneur est de 83 % molaire de $Fe_{2.2}C$ et 17 % molaire de $Fe_5C_2$. Les résultats de VSM à 300 K indiquent en outre une aimantation à saturation Ms d'environ 151 emu/g.

Exemple 4 - synthèse de nanoparticules de carbure de fer de 15,0 nm contenant 82 % de la structure cristalline $Fe_{2.2}C$

Le protocole général ci-dessus est appliqué avec les paramètres suivants : les nanoparticules de $Fe^0$ obtenues dans l'Exemple 2 ci-avant (1 mmol Fe ; 100 mg) sont placées dans une bouteille Fischer-Porter et redispersées dans le mésitylène (20 mL). La bouteille Fischer-Porter est fermée et sortie de la boite à gants, purgée de son argon puis mise sous pression de monoxyde de carbone (2 bars) et d'hydrogène (2 bars). Le mélange est ensuite agité vigoureusement à 150 °C pendant 140 h.

Les nanoparticules obtenues, nommées ci-après FeCNP2, sont caractérisées. Les résultats obtenus par TEM, DRX et spectroscopie Môssbauer sont montrés sur la figure 4, respectivement en (a), (b) et (c). On observe que les nanoparticules sont sphériques, monodisperses, de diamètre D = 15,0 nm +/- 0,9 nm, et qu'elles comprennent un cœur monocristallin de $Fe_{2,2}C$. Il ressort de la spectroscopie Môssbauer que leur teneur molaire est de 82 % de $Fe_{2.2}C$ et 18 % de $Fe_5C_2$. Les résultats de VSM à 300 K indiquent en outre une aimantation à saturation Ms d'environ 170 emu/g.

Exemples 5 - 7

Des nanoparticules de carbure de fer sont préparées selon le protocole général ci-dessus, pour des temps de carburation différents.

Les paramètres opératoires mis en œuvre sont les suivants : les nanoparticules de $Fe^0$ obtenues dans l'Exemple 1 ou l'Exemple 2 ci-avant (1 mmol Fe ; 100 mg) sont placées dans la bouteille Fischer-Porter et redispersées dans le mésitylène (20 mL). La bouteille Fischer-Porter est mise sous pression de monoxyde de carbone (2 bars) et d'hydrogène (2 bars). Le mélange est ensuite agité vigoureusement à 150 °C pendant une durée de carburation t.

Les nanoparticules obtenues sont caractérisées. Il est vérifié par analyse DRX que leur cœur est constitué exclusivement de $Fe_{2,2}C$. Leur teneur molaire en $Fe_{2,2}C$ est par ailleurs déterminée par spectroscopie Môssbauer.

Les caractéristiques des nanoparticules ainsi préparées et les paramètres opératoires mis en œuvre pour leur préparation sont récapitulés dans le tableau 1 ci-après.

Tableau 1 - caractéristiques de nanoparticules conformes à l'invention et paramètres opératoires pour leur préparation

| Référence | Nanoparticules de $Fe^0$ | Durée de carburation t (h) | Diamètre des nanoparticules (nm) |
|---|---|---|---|
| FeCNP3 | FeNP1 | 72 | 12,1 |
| FeCNP4 | FeNP1 | 96 | 13,1 |

(suite)

| Référence | Nanoparticules de $Fe^0$ | Durée de carburation t (h) | Diamètre des nanoparticules (nm) |
|---|---|---|---|
| FeCNP5 | FeNP1 | 144 | 13,3 |

Exemple comparatif 1 - synthèse de nanoparticules de carbure de fer de 9,7 nm contenant 59 % de la structure cristalline $Fe_{2.2}C$

Le protocole général ci-dessus est appliqué avec les paramètres suivants : les nanoparticules de Fe0 obtenues dans l'Exemple 1 ci-avant (1 mmol Fe ; 100 mg) sont placées dans une bouteille Fischer-Porter et redispersées dans le mésitylène (20 mL). La bouteille Fischer-Porter est mise sous pression de monoxyde de carbone (2 bars) et d'hydrogène (2 bars). Le mélange est ensuite agité vigoureusement à 150 °C pendant 24 h.

Les nanoparticules obtenues, nommées ci-après FeCcomp2, sont caractérisées. Les résultats obtenus par TEM, DRX et spectroscopie Môssbauer sont montrés sur la figure 5, respectivement en (a), (b) et (c). On observe que les nanoparticules sont sphériques, monodisperses, de diamètre D = 9,7 nm +/- 0,5 nm, et qu'elles comprennent un cœur monocristallin de $Fe_{2,2}C$. Il ressort de la spectroscopie Môssbauer que leur teneur molaire est de 59 % de $Fe_{2.2}C$, 16 % de $Fe_5C_2$, 21 % de $Fe^0$ et 4 % de phase paramagnétique (amorphe). Les résultats de VSM à 300 K indiquent en outre une aimantation à saturation Ms d'environ 150 emu/g.

Exemples comparatifs 2 à 9

Des nanoparticules de carbure de fer sont préparées selon le protocole général ci-dessus, pour des temps de carburation inférieurs à 72 h.

Les paramètres opératoires mis en œuvre sont les suivants : les nanoparticules de $Fe^0$ obtenues dans l'Exemple 1 ou l'Exemple 2 ci-avant (1 mmol Fe ; 100 mg) sont placées dans la bouteille Fischer-Porter et redispersées dans le mésitylène (20 mL). La bouteille Fischer-Porter est mise sous pression de monoxyde de carbone (2 bars) et d'hydrogène (2 bars). Le mélange est ensuite agité vigoureusement à 150 °C pendant une durée de carburation t.

Les nanoparticules obtenues sont caractérisées. Il est établi par analyse DRX que leur cœur est constitué de $Fe_{2,2}C$ ou d'un mélange de $Fe_{2,2}C$ et de $Fe^0$. Leur teneur molaire en $Fe_{2,2}C$ est par ailleurs déterminée par spectroscopie Môssbauer.

Les caractéristiques des nanoparticules ainsi préparées et les paramètres opératoires mis en œuvre pour leur préparation sont récapitulés dans le tableau 2 ci-dessous.

Tableau 2 - caractéristiques de nanoparticules comparatives et paramètres opératoires pour leur préparation

| Référence | Nanoparticules de $Fe^0$ | Durée de carburation t (h) | Composition du cœur | % molaire de $Fe_{2,2}C$ |
|---|---|---|---|---|
| FeCcomp3 | FeNP1 | 48 | $Fe_{2,2}C$ | - |
| FeCcomp4 | FeNP1 | 16 | $Fe_{2,2}C + Fe^0$ | - |
| FeCcomp5 | FeNP1 | 8 | $Fe_{2,2}C + Fe^0$ | - |
| FeCcomp6 | FeNP1 | 4 | $Fe_{2,2}C + Fe^0$ | - |
| FeCcomp7 | FeNP1 | 2 | $Fe_{2,2}C + Fe^0$ | - |
| FeCcomp8 | FeNP2 | 48 | $Fe_{2,2}C + Fe^0$ | 67 |
| FeCcomp9 | FeNP2 | 15 | $Fe_{2,2}C + Fe^0$ | - |
| FeCcomp10 | FeNP2 | 4 | $Fe_{2,2}C + Fe^0$ | - |

## D/ Synthèse de nanoparticules de carbure de fer recouvertes de nickel

Protocole général

En boîte à gant, les nanoparticules de carbure de fer sont placées dans une bouteille Fischer-porter et redispersées dans le mésitylène. De l'acide palmitique est ajouté afin de faciliter la redispersion des nanoparticules et améliorer leur stabilité en solution. Le précurseur de nickel $Ni(acac)_2$ (Bis(acétylacétonate)Nickel) préalablement solubilisé dans le mésitylène, est introduit dans la bouteille Fischer-Porter. La bouteille est fermée et sortie de la boite à gant, puis

passée aux ultrasons pendant 15 s à 10 min (préférablement 1 min). Le mélange est agité vigoureusement sous argon à 120-180 °C (préférablement 150 °C) pendant 10 min à 4 h (préférablement 1 h) afin d'homogénéiser la solution. Enfin, la bouteille Fischer-Porter est mise sous pression d'hydrogène comprise entre 1 et 10 bars (préférablement 3 bars). Le mélange est agité vigoureusement à 150 °C pendant une durée comprise entre 1 et 48 h (préférablement pendant 24 h).

Une fois la réaction terminée, la bouteille Fischer-Porter est retirée du bain d'huile et laissée à refroidir sous agitation. Une fois à température ambiante, elle est passée aux ultrasons pendant 15 s à 10 min (préférablement 1 min) puis entrée en boîte à gant. Les nanoparticules sont lavées, par lavage magnétique, trois fois au toluène puis séchées sous rampe à vide. Les nanoparticules obtenues sont analysées en TEM, DRX, VSM, microscopie électronique en transmission à haute résolution (HRTEM) et microscopie électronique à balayage par transmission couplée à la spectroscopie à rayons X à dispersion d'énergie (STEM-EDX).

Exemple 8

Le protocole général ci-dessus est appliqué à partir des nanoparticules de carbure de fer obtenues à l'Exemple 4 ci-avant, avec les paramètres opératoires décrits ci-après.

Les nanoparticules (1 mmol ; 80 mg) sont redispersées dans le mésitylène (15 mL). De l'acide palmitique (0,5 mmol ; 128,4 mg) est ajouté. Le précurseur de nickel $Ni(acac)_2$ (0,5 mmol ; 129,3 mg), préalablement solubilisé dans le mésitylène (10 mL + 5 mL rinçage), est introduit dans la bouteille Fischer-Porter. Cette dernière est fermée, sortie de la boite à gants puis passée aux ultrasons pendant 1 min. Le mélange est agité vigoureusement sous argon à 150 °C pendant 1 h. Enfin, la bouteille Fischer-Porter est mise sous pression d'hydrogène (3 bars). Le mélange est agité vigoureusement à 150 °C pendant 24 h.

Une fois à température ambiante, la bouteille Fischer-Porter est passée aux ultrasons pendant 1 min.

Les nanoparticules obtenues, nommées ci-après FeC@Ni1, sont caractérisées. Les résultats obtenus par TEM, DRX et STEM-EDX sont montrés sur la figure 6, respectivement en (a), (b) et (c1) (image STEM) brute), (c2) (image STEM-EDX ciblée sur le fer) et (c3) (image STEM-EDX ciblée sur le nickel). On observe que les nanoparticules sont sphériques, monodisperses, de diamètre D = 15,2 nm +/- 1,1 nm. L'analyse DRX confirme la présence du cœur cristallin constitué de $Fe_{2,2}C$, et montre la croissance de nickel sous forme métallique à la surface des nanoparticules, ainsi que l'absence d'oxydes de nickel. L'analyse STEM-EDX confirme que le fer est concentré dans le cœur des nanoparticules, et que le nickel est quant à lui présent en surface.

**E/ Mesures d'hyperthermie par induction magnétique**

Protocole général

En boîte à gant, 10 mg de nanoparticules sont placées dans un tube auquel sont ajoutés 0,5 ml de mésitylène. Le tube est sorti de la boîte à gant et traité 1 min aux ultrasons afin d'obtenir une solution colloïdale de nanoparticules. Le tube est ensuite placé dans un calorimètre contenant 2 mL d'eau déionisée. Le calorimètre est exposé à un champ magnétique alternatif (100 kHz, amplitude ajustable entre 0 et 47 mT) pendant 40 secondes et l'échauffement de l'eau est mesuré par deux sondes optiques de température. L'élévation de température est déterminée par la pente moyenne de la fonction $\Delta T/\Delta t$. Pour finir, le SAR (Specific Absorption Rate) est calculé grâce à l'équation suivante :

$$SAR = \frac{\sum_i C_{pi} m_i}{m_{Fe}} \frac{\Delta T}{\Delta t}$$

dans laquelle : $C_{pi}$ représente la capacité calorifique du composé i ($C_p$ = 449 J $kg^{-1}K^{-1}$ pour les nanoparticules de Fe, $C_p$ = 1750 J $kg^{-1}K^{-1}$ pour le mésitylène, $C_p$ = 4186 J $kg^{-1}K^{-1}$ pour l'eau et $C_p$ = 720 J $kg^{-1}K^{-1}$ pour le verre) ; $m_i$ représente la masse du composé i ; $m_{Fe}$ représente la masse de fer dans l'échantillon.

Expérience 1

Une première expérience est réalisée pour les nanoparticules de $Fe^0$ de l'Exemple 2 (FeNP2) et les nanoparticules de carbure de fer de l'Exemple 4 (FeCNP2).

A titre comparatif, sont également testés en parallèle des nanoparticules de carbure de fer préparées conformément au protocole décrit dans la publication de Meffre et al, 2012, Nanoletters, 4722-4728, de composition 43 % $Fe_{2.2}C$, 43 % $Fe_5C_2$, 14 % d'espèces paramagnétiques (FeCcomp1).

Les résultats obtenus sont en outre comparés à ceux présentés pour des nanoparticules d'oxyde de fer dans la publication de Pellegrino et al, 2014, J. Mater. Chem. B, 4426-4434, décrites pour présenter un SAR élevé (FeONP).

Les résultats obtenus sont montrés sur la figure 7.

On observe que les nanoparticules conformes à l'invention FeCNP2 présentent des performances d'hyperthermie largement supérieures à celles des autres nanoparticules.

Expérience 2

Les SAR maximaux obtenus à 100 kHz, pour un champ magnétique d'amplitude 47 mT, pour les différentes nanoparticules ci-dessous, sont indiqués dans le tableau 3 ci-après.

Tableau 3 - SAR maximaux obtenus à 100 kHz pour des nanoparticules conformes à l'invention et des nanoparticules comparatives

| Nanoparticule | SARmax (W/g) |
|---|---|
| FeNP1 | 425 |
| FeCcomp6 | 1070 |
| FeCcomp5 | 450 |
| FeCcomp4 | 95 |
| FeCcomp3 | 890 |
| FeCcomp2 | 45 |
| FeCNP1 | 1630 |
| FeCNP3 | 1485 |
| FeNP2 | 1220 |
| FeCcomp9 | 660 |
| FeCcomp8 | 100 |
| FeCNP2 | 3300 |

On observe que les nanoparticules conformes à l'invention présentent toutes des SAR bien supérieurs à ceux des nanoparticules comparatives, et à ceux des nanoparticules de fer zéro-valent ayant servi à leur préparation.

Expérience 3

Les nanoparticules de fer zéro-valent FeNP1, les nanoparticules de carbure de fer conformes à l'invention (FeCNP1, FeCNP3, FeCNP4 et FeCNP5) et les nanoparticules de carbure de fer comparatives (FeCcomp2, FeCcomp3, FeCcomp4, FeCcomp6, FeCcomp7), obtenues à partir de ces nanoparticules FeNP1, sont soumises au protocole de test ci-avant. Le SAR est mesuré pour différentes amplitudes du champ magnétique.

Les résultats obtenus sont montrés sur la figure 8.

On observe que non seulement les nanoparticules selon l'invention présentent des SAR bien plus élevées que les nanoparticules comparatives, mais qu'en outre, leurs performances d'hyperthermie s'exercent à des champs magnétiques de faible amplitude, dès 25 mT pour certaines. Ces performances sont élevées à partir d'environ 38 mT pour toutes les nanoparticules conformes à l'invention.

Expérience 4

Les nanoparticules de fer zéro-valent FeNP2, les nanoparticules de carbure de fer conformes à l'invention (FeCNP2) et les nanoparticules de carbure de fer comparatives (FeCcomp8, FeCcomp9, FeCcomp10), obtenues à partir de ces nanoparticules FeNP2, sont soumises au protocole de test ci-avant. Le SAR est mesuré pour différentes amplitudes du champ magnétique.

Les résultats obtenus sont montrés sur la figure 9.

On observe que non seulement les nanoparticules selon l'invention présentent un SAR bien plus élevée que les nanoparticules comparatives, mais qu'en outre, leurs performances d'hyperthermie s'exercent à des champs magnétiques de faible amplitude, et sont particulièrement élevés dès 25 mT environ.

Expérience 5 - influence de la taille des nanoparticules

Les SAR des nanoparticules conformes à l'invention FeCNP1 (diamètre environ 13 nm) et FeCNP2 (diamètre environ 15 nm) sont soumises au protocole de test ci-avant.

Les résultats obtenus sont montrés sur la figure 10.

On observe que les deux types de nanoparticules présentent un pouvoir de chauffe élevé, les nanoparticules de taille d'environ 15 nm étant cependant plus performantes que les nanoparticules de taille d'environ 13 nm.

Expérience 6

Les SAR des nanoparticules conformes à l'invention FeCNP2 et FeC@Ni sont soumises au protocole de test ci-avant.

Les résultats obtenus sont montrés sur la figure 11.

On observe que les nanoparticules recouvertes de nickel présentent un pouvoir de chauffe sensiblement équivalent à celui des nanoparticules non recouvertes pour des champs magnétiques au-delà de 40 mT.

**F/ Catalyse de la réaction de Sabatier par induction magnétique**

Protocole général

Les nanoparticules de carbure de fer sont mises en œuvre pour catalyser la réaction de Sabatier, selon le schéma réactionnel :

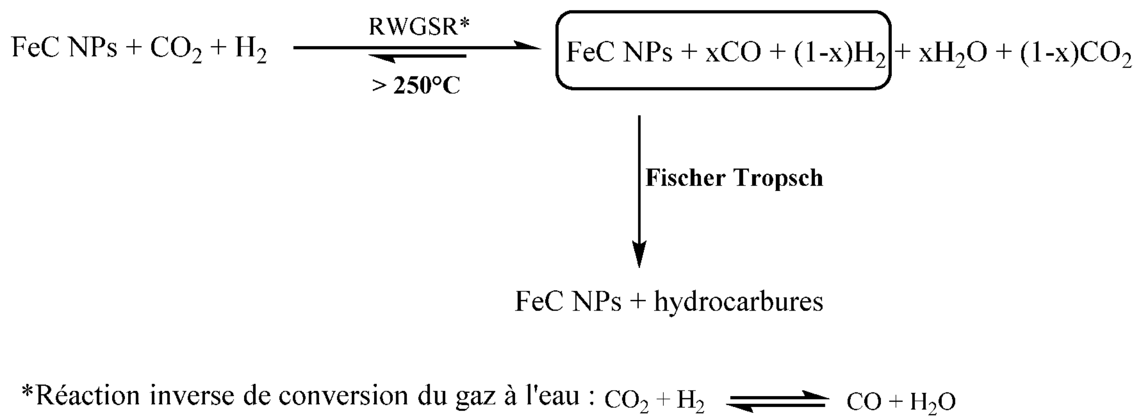


dans lequel FeC NPs représente les nanoparticules de carbure de fer.

A cet effet, en boîte à gant, le catalyseur sous forme de poudre (10 mg) est placé dans une bouteille Fischer-Porter munie au niveau de sa tête d'un manomètre afin de suivre la variation de pression au cours de la réaction, sans aucun solvant. La bouteille Fischer-Porter est fermée, sortie de boîte à gant, vidée de son argon et mise sous pression de $CO_2$ (1 équivalent ; 0,8 bar : de -1 bar à-0,2 bar) et de $H_2$ (4 équivalent ; 3,2 bar : de -0,2 bar à 3 bar). La bouteille Fischer-Porter est ensuite exposée à un champ magnétique alternatif (300 kHz, amplitude ajustable entre 0 et 64 mT) pendant 8 h. A la fin de la réaction, la phase gazeuse est analysée par spectrométrie de masse afin d'identifier les composés formés.

Expérience 1

Dans cette expérience sont mises en œuvre les nanoparticules de carbure de fer conformes à l'invention FeCNP2. Les résultats obtenus, en termes d'une part de taux de conversion du $CO_2$, et d'autre part de rendement en hydrocarbure(s), en fonction de l'amplitude du champ magnétique appliqué, sont montrés sur la figure 12. Ces résultats montrent que le taux de conversion du $CO_2$ est proche de 100 % à des amplitudes de champ magnétique même inférieures à 30 mT. Le rendement en hydrocarbure(s) est très élevé, de l'ordre de 80 % au-delà de 30 mT, et ce sans avoir eu recours à dopage par un autre élément tel que le cobalt ou le ruthénium.

Le spectre de masse obtenu pour la phase gazeuse à 30 mT est montré sur la figure 13. On y observe que le

méthane ($CH_4$) est le composé très majoritairement formé.

Ainsi, les nanoparticules de carbure de fer conformes à l'invention présentent une très bonne activité catalytique à un champ supérieur ou égal à 30 mT. La sélectivité en méthane est également très élevée, d'environ 80 %.

Expérience 2

Dans cette expérience sont mises en œuvre les nanoparticules de carbure de fer recouvertes de nickel conformes à l'invention FeC@Ni, et les nanoparticules de carbure de fer conformes à l'invention FeCNP2.

Les protocoles opératoires diffèrent de celui décrit précédemment en ce qui concerne la durée d'application du champ magnétique et l'amplitude de ce dernier.

Les paramètres opératoires exacts et les résultats associés, en termes de taux de conversion du $CO_2$, de rendement en hydrocarbure(s) et de sélectivité par rapport au méthane, sont indiqués dans le tableau 4 ci-après.

Tableau 4 - Paramètres opératoires et résultats de la catalyse de la réaction de Sabatier par induction magnétique de nanoparticules conformes à l'invention

| Nanopart. | Durée d'induction et amplitude du champ | Taux de conversion du $CO_2$ (%) | Rendement en hydrocarbure (%) | Sélectivité / méthane (%) |
|---|---|---|---|---|
| FeC@Ni | 3 h à 64 mT | 84 | 74 | 97 |
| FeC@Ni | activation 5 s à 64 mT puis 8 h à 25 mT | 36 | 27 | > 99 |
| FeCNP2 | 8 h à 64 mT | > 98 | 76 | 80 |

On en déduit que :

- pour les nanoparticules recouvertes de nickel, la réaction est quasi quantitative en 3 h à 64 mT, et la sélectivité en méthane est quasi-totale. Il ressort en outre du spectre de masse (non montré) que le dioxyde de carbone est transformé quantitativement d'une part en méthane et d'autre part en une faible quantité de monoxyde de carbone ;

- à même amplitude de champ magnétique, les nanoparticules recouvertes de nickel permettent d'atteindre des rendements similaires en hydrocarbures, et une sélectivité en méthane supérieure, par rapport aux nanoparticules non recouvertes, et ce en des durées bien moindres ;

- pour les nanoparticules recouvertes de nickel, après activation de quelques secondes à 64 mT, puis 8 h à 25 mT, on observe une activité catalytique, bien qu'à la valeur de 25 mT, le SAR des nanoparticules soit nul (cf. figure 11). Ceci démontre qu'il est possible de catalyser la réaction de Sabatier avec une faible consommation d'énergie, par une première phase très courte d'activation à champ magnétique élevé, suivie d'une phase à champ magnétique de beaucoup plus faible amplitude.

Ainsi, dans le cas des nanoparticules de carbure de fer recouvertes de nickel, il est possible d'activer la réaction à fort champ (64 mT) pendant quelques secondes et ensuite de travailler à faible champ (25 mT) pendant plusieurs heures. La réaction étant exothermique, une fois initiée, il est avantageusement possible de l'entretenir à faible coût énergétique.

A titre comparatif, le même protocole a été appliqué pour des nanoparticules de carbure de fer de l'art antérieur (contenant 43 % de $Fe_{2,2}C$, préparées selon la publication de Meffre et al., 2012, Nanoletters, 12, 4722-4728). Il n'a été observé aucune activité catalytique pour ces nanoparticules.

Expérience 3

Dans cette expérience sont mises en œuvre les nanoparticules de carbure de fer conformes à l'invention FeCNP1. L'amplitude du champ magnétique est fixée à 40,2 mT.

Le spectre de masse obtenu pour la phase gazeuse à l'issue de la réaction est montré sur la figure 14. On en déduit un taux de conversion du $CO_2$ d'environ 55 %, et un rendement en hydrocarbures d'environ 37 %, le méthane étant le composé principalement formé.

A l'issue de la réaction, les nanoparticules sont analysées par DRX. Le diffractogramme obtenu est montré sur la figure 15. Lorsqu'on le compare au diffractogramme RX des nanoparticules avant catalyse, montré sur la figure 3(c),

on constate que les nanoparticules n'ont subi qu'une très légère modification de leur structure lors de la réaction. Le catalyseur comprenant les nanoparticules peut être réutilisé plusieurs fois sans perte d'activité.

La description ci-avant démontre clairement que les nanoparticules de carbure de fer conformes à l'invention présentent la capacité de catalyser la réaction de Sabatier par induction magnétique. Pour les nanoparticules testées, on obtient une conversion totale du dioxyde de carbone à 30 mT et 300 kHz, ceci sans mettre en œuvre de catalyseur supplémentaire. Aux conditions testées, on n'observe que peu de modification du catalyseur.

**G/ Mesures comparatives d'hyperthermie par induction magnétique**

Synthèse de nanoparticules de carbure de fer

Il est préparé des nanoparticules de carbure de fer avec les différents temps de carburation suivants, à partir du même lot de nanoparticules de $Fe^0$ de 12,5 nm, obtenues à l'Exemple 2 ci-avant.

Le protocole général est celui décrit dans l'Exemple 4 ci-avant, seul le temps de carburation variant, et étant égal à 48 h (nanoparticules NP48), 96 h (nanoparticules NP96) ou 140 h (nanoparticules NP140).

Les spectres Môssbauer pour chacune de ces nanoparticules sont montrés sur la figure 16. On en déduit les compositions pour les nanoparticules indiquées dans le tableau 5 ci-après. Les nanoparticules NP96 et NP140 sont conformes à l'invention, alors que les nanoparticules NP48 ne sont pas conformes à l'invention, car présentant une teneur molaire en $Fe_{2,2}C$ insuffisante.

Les propriétés d'hyperthermie de ces nanoparticules ont été analysées comme décrit dans l'Exemple E/ ci-avant. Les courbes présentant, pour chacune, le SAR en fonction de l'amplitude du champ magnétique, sont montrées sur la figure 17. Les SAR maximaux pour chacune sont indiqués dans le tableau 5 ci-après.

Tableau 5 - Composition et SAR maximal pour des nanoparticules de carbure de fer conformes (NP96, NP140) ou non conformes à l'invention

| Nanoparticule | Composition | SARmax (W/g) |
|---|---|---|
| $Fe^0$ | - | 650 |
| NP48 | 54% $Fe_{2,2}C$, 23% $Fe_5C_2$, 18% Fe(0), 5% autres | 460 |
| NP96 | 72% $Fe_{2,2}C$, 24% $Fe_5C_2$, 4% Fe(0) | 2120 |
| NP140 | 83% $Fe_{2,2}C$, 17% $Fe_5C_2$ | 3220 |

On observe que les nanoparticules conformes à l'invention NP96 et NP140 présentent des performances d'hyperthermie largement supérieures à celles de autres nanoparticules (NP48).

**H/ Synthèse de nanoparticules de carbure de fer avec élimination d'eau**

Protocole général

En boite à gants, les nanoparticules de $Fe^0$ sont placées dans une bouteille Fischer-Porter et redispersées dans le mésitylène. La partie supérieure de la bouteille Fischer-Porter utilisée possède une cartouche en verre greffée à la paroi (bouteille Fischer-Porter fabriquée par la société Avitec) dans laquelle est introduit du tamis moléculaire préalablement activé (1,5 g environ). Le tamis moléculaire n'est pas en contact avec la solution de nanoparticules, et reste à température modérée. La bouteille de Fischer-Porter est fermée et sortie de la boite à gants, purgée de son argon puis mise sous pression de monoxyde de carbone (entre 1 et 10 bars) et d'hydrogène (entre 1 et 3 bars) afin d'obtenir une surpression de 3 bars dans la bouteille. Le mélange est ensuite agité vigoureusement à 120-180 °C pendant 1 min à 200 h.

Une fois la réaction terminée, la bouteille de Fischer-Porter est retirée du bain d'huile et laissée refroidir sous agitation. Une fois à température ambiante, elle est entrée en boite à gants et dégazée. Les nanoparticules sont lavées, par lavage magnétique, 3 fois au toluène puis séchées sous rampe à vide. La poudre noire obtenue est analysée en TEM, DRX, VSM et analyse élémentaire.

Exemple 9 - à partir de nanoparticules de $Fe^0$ de 12.5 nm

En boite à gants, les nanoparticules de $Fe^0$ (12.5 nm ; 1 mmol Fe ; 100 mg), préparées à l'Exemple 2, sont

placées dans la bouteille Fischer-Porter et redispersées dans le mésitylène (20 mL). La bouteille de Fischer-Porter est mise sous pression de monoxyde de carbone (2 bars) et d'hydrogène (2 bars). Le mélange est ensuite agité vigoureusement à 150 °C pendant 40 h.

A titre de comparaison, la même expérience est réalisée en parallèle sans tamis moléculaire, sur des durées de 48 h et 140 h.

Après un temps de réaction de 16 h, un échantillon des nanoparticules est prélevé et analysé par DRX. On n'observe plus aucune présence de Fe(0). Pour la même expérience réalisée en parallèle sans tamis moléculaire, on obtient après16 h une composition d'environ 50 % de Fe(0) et 50 % de $Fe_{2,2}C$. Ceci confirme que la mise en œuvre du tamis moléculaire, permettant d'éliminer l'eau au fur et à mesure de sa formation dans la réaction, a pour effet d'accélérer la réaction de carburation.

Après 40 h de réaction, on obtient pour l'expérience avec tamis moléculaire des nanoparticules comportant une teneur molaire, déterminée par analyse Môssbauer, supérieure à 75 % de $Fe_{2.2}C$.

Pour chacune des expériences avec et sans tamis moléculaire, les propriétés d'hyperthermie des nanoparticules obtenues sont analysées comme décrit dans l'Exemple E/ ci-avant. Les courbes présentant, pour chacune, le SAR en fonction de l'amplitude du champ magnétique, sont montrées sur la figure 18. On observe que pour l'expérience avec tamis moléculaire (NP40TM), on obtient en 40 h des performances aussi élevées qu'en 140 h en l'absence de tamis moléculaire (NP140S).

Exemple 10 - à partir de nanoparticules de $Fe^0$ de 9.0 nm

En boite à gants, des nanoparticules de $Fe^0$ (9.0 nm ; 1 mmol Fe ; 100 mg) (d'origine commerciale) sont placées dans la bouteille Fischer-Porter et redispersées dans le mésitylène (20 mL). La bouteille de Fischer-Porter est mise sous pression de monoxyde de carbone (2 bars) et d'hydrogène (2 bars). Le mélange est ensuite agité vigoureusement à 150 °C pendant 24 h. cette expérience est réalisée en double.

A titre de comparaison, la même expérience est réalisée en parallèle sans tamis moléculaire, sur des durées de 24 h et 120 h.

Après 24 h de réaction, on obtient pour l'expérience avec tamis moléculaire des nanoparticules comportant une teneur molaire, déterminée par analyse Môssbauer, supérieure à 75 % de $Fe_{2.2}C$.

Pour chacune des expériences avec et sans tamis moléculaire, les propriétés d'hyperthermie des nanoparticules obtenues sont analysées comme décrit dans l'Exemple E/ ci-avant. Les courbes présentant, pour chacune, le SAR en fonction de l'amplitude du champ magnétique, sont montrées sur la figure 19. On observe que pour les expériences avec tamis moléculaire (NP24TM), on obtient en 24 h des performances aussi élevées qu'en 120 h en l'absence de tamis moléculaire (NP120S).

**I/ Synthèse de nanoparticules de carbure de fer recouvertes de nickel supportées sur Siralox®**

Synthèse de Siralox® dopé au ruthénium

En boite à gants, le Siralox® (800 mg) est ajouté à une solution orange de Ru(COD)(COT) (120,0 mg, 0,38 mmol) dans le mésitylène (5 mL). Le mélange est agité sous argon à température ambiante pendant 1 h, puis mis sous pression de dihydrogène (3 bar) et agité à température ambiante pendant 24 h. A la fin de la réaction, la poudre est récupérée par décantation et lavée trois fois au toluène (3x 5 mL). Le Siralox® dopé au ruthénium Ru est alors séché sous vide. D'après les analyses élémentaires, il contient 1 % en poids de Ru.

Synthèse des nanoparticules supportées sur Siralox®

En boite à gants, les nanoparticules magnétiques (FeC@Ni1 de l'Exemple 8 ou FeCNP2 de l'Exemple 4) (100 mg, soit environ 75 mg de Fe) sont dispersées dans le toluène (5 mL). Le Siralox® (Sir) ou le Siralox® dopé au Ru comme décrit ci-avant (RuSir) (700 mg) est ajouté à la solution, et le mélange résultant est exposé aux ultrasons (hors boite à gants) pendant environ 1 min. La bouteille de Fischer-Porter est entrée à nouveau en boite à gants, et le surnageant est éliminé après décantation. La poudre noire obtenue est séchée sous vide. On obtient un chargement à environ 10 % en poids de Fe sur le Siralox®.

**J/ Catalyse de la réaction de Sabatier en réacteur à flux continus**

Protocole général

En boite à gants, le catalyseur supporté (800 mg) est chargé sous forme de poudre dans un réacteur en verre

(1 mL, 1 cm x 0,8 cm). Le réacteur est ensuite connecté à l'installation de catalyse, placé au centre d'une bobine d'induction, et alimenté par un flux de $H_2$ et $CO_2$ contrôlé par des flux massiques (rapport $H_2/CO_2$ = 4, stœchiométrique). Pour un test standard, le débit total est fixé à 25 $mL.min^{-1}$ (Vitesse Volumétrique Horaire VVH = 1500 $h^{-1}$). Le réacteur, placé au centre de la bobine d'induction, est exposé à des champs magnétiques alternatifs de fréquence 300 kHz et d'amplitudes comprises entre 0 et 64 mT. En sortie de réacteur, les gaz sont analysés en direct par GC-MS.

Exemple 11 - Nanoparticules FeC@Ni1 supportées sur Sir

Les taux de conversion de $CO_2$, de formation de $CH_4$ et de formation de CO, en fonction de l'amplitude du champ magnétique, mesurés après 2 heures de réaction, sont montrés sur la figure 20. On observe qu'il se produit la conversion du $CO_2$, à un taux de conversion élevé. En outre, du point de vue de la formation de $CH_4$, l'amplitude optimale se trouve aux environs de 40 mT (zone grisée sur la figure). Elle est associée à un rendement en $CH_4$ d'environ 14 %. Au-delà, plus l'amplitude du champ magnétique augmente, et plus le rendement en CO augmente et celui en $CH_4$ diminue.

Le chromatogramme en phase gazeuse obtenu en sortie du réacteur, pour l'amplitude de 40 mT, est montré sur la figure 21. Il confirme la présence de $CH_4$ en sortie du réacteur.

Exemple 12 - Nanoparticules FeCNP2 supportées sur RuSir

Dans cet exemple, sont mises en œuvre les nanoparticules selon l'invention FeCNP2, sur le support Siralox® dopé au ruthénium. Une image de microscopie électronique d'un grain de poudre obtenu comme décrit à l'Exemple I/ est montré sur la figure 22. Elle confirme la présence des nanoparticules de carbure de fer à la surface du grain, sous forme de billes noires dont l'une est indiquée par la flèche sur la figure.

Les taux de conversion de $CO_2$, de sélectivité vis-à-vis du $CH_4$ et de formation de CO, en fonction de l'amplitude du champ magnétique, mesurés après 2 heures de réaction, sont montrés sur la figure 23. On observe un taux de conversion élevé du $CO_2$, dès une amplitude du champ magnétique de 25 mT. En outre, la sélectivité vis-à-vis de la formation de $CH_4$ est particulièrement forte, proche de 100%. L'amplitude optimale se trouve dans la plage de 25 à 30 mT (zone grisée sur la figure). Elle est associée à un rendement en $CH_4$ d'environ 86 %.

Le chromatogramme en phase gazeuse obtenu en sortie du réacteur, pour l'amplitude de 28 mT, est montré sur la figure 24. Il confirme la présence largement majoritaire de $CH_4$ en sortie du réacteur.

Ces résultats démontrent que les nanoparticules conformes à l'invention permettent de réaliser la catalyse de la réaction de Sabatier, dans un mode opératoire à flux continu, avec de forts rendements et une sélectivité totale pour la formation du méthane.

Des résultats similaires sont obtenus pour des débits totaux dans la plage de 25 à 125 mL/min.

L'expérience est poursuivie pendant 150 h sous champ magnétique de 28 mT, en évaluant régulièrement les taux de conversion de $CO_2$, de sélectivité vis-à-vis du $CH_4$ et de formation de CO, ainsi que la température dans le réacteur. Les courbes obtenues pour ces différents paramètres, en fonction du temps, sont montrées sur la figure 25. Elles confirment que la puissance de chauffe des nanoparticules selon l'invention, et l'activité catalytique, sont stables après une durée aussi longue que 150 heures de réaction en flux continu. Ceci s'avère d'autant plus avantageux que le fonctionnement de l'inducteur de champ magnétique a été interrompu à plusieurs reprises lors de l'expérience, démontrant une aptitude des nanoparticules selon l'invention à fonctionner en intermittence.

## Revendications

1. Nanoparticule de carbure de fer, **caractérisée en ce qu'**au moins 70 % des atomes de fer qu'elle comporte sont présents dans une structure cristalline de $Fe_{2,2}C$.

2. Nanoparticule de carbure de fer selon la revendication 1, **caractérisée en ce qu'**au moins 80 % des atomes de fer qu'elle comporte sont présents dans une structure cristalline de $Fe_{2,2}C$

3. Nanoparticule de carbure de fer selon l'une quelconque des revendications 1 à 2, de taille comprise entre 1 et 20 nm, de préférence comprise entre 10 et 16 nm.

4. Nanoparticule de carbure de fer selon l'une quelconque des revendications 1 à 2, de taille égale à 15 nm $\pm$ 1 nm.

5. Nanoparticule de carbure de fer selon l'une quelconque des revendications 1 à 4, recouverte sur au moins une partie de sa surface d'un revêtement d'un métal catalytique.

6. Nanoparticule de carbure de fer selon la revendication 5, dans laquelle ledit métal catalytique est choisi parmi le nickel, le ruthénium, le cobalt, le cuivre, le zinc, le platine, le palladium, le rhodium, le manganèse, le molybdène, le tungstène, le vanadium, l'iridium, l'or, ou l'un quelconque de leurs alliages, seuls ou en mélange.

7. Nanoparticule de carbure de fer selon l'une quelconque des revendications 1 à 6, susceptible d'être obtenue par une étape de carburation de nanoparticule de fer zéro-valent par mise en présence de ladite nanoparticule de fer zéro-valent avec un mélange gazeux de dihydrogène et de monoxyde de carbone.

8. Nanoparticule de carbure de fer selon l'une quelconque des revendications 1 à 7, supportée sur un support solide, ledit support solide étant le cas échéant dopé par un métal catalytique.

9. Procédé de préparation de nanoparticules de carbure de fer selon l'une quelconque des revendications 1 à 8, **caractérisé en ce qu'**il comprend une étape de carburation de nanoparticules de fer zéro-valent par mise en présence desdites nanoparticules de fer zéro-valent avec un mélange gazeux de dihydrogène et de monoxyde de carbone.

10. Procédé de préparation selon la revendication 9, selon lequel ladite étape de carburation est réalisée à une température comprise entre 120 et 300 °C, de préférence entre 120 et 180 °C.

11. Procédé de préparation selon l'une quelconque des revendications 9 à 10, selon lequel ladite étape de carburation est réalisée pendant une durée comprise entre 72 et 200 h.

12. Procédé de préparation selon l'une quelconque des revendications 9 à 10, selon lequel ladite étape de carburation comprend l'élimination de l'eau formée lors de la réaction desdites nanoparticules de fer zéro-valent et dudit mélange gazeux, au fur et à mesure de sa formation, et ladite étape de carburation est de préférence réalisée pendant une durée comprise entre 24 et 60 h.

13. Procédé de préparation selon l'une quelconque des revendications 9 à 12, comprenant une étape préalable de préparation des nanoparticules de fer zéro-valent par décomposition d'un précurseur organométallique répondant à la formule générale (I) :

$$Fe(NR^1R^2)(NR^3R^4) \qquad (I)$$

dans laquelle $R^1$, $R^2$, $R^3$ et $R^4$, identiques ou différents, représentent chacun un groupement alkyle, aryle, triméthylsilyle ou triméthylalkyle,
en présence de dihydrogène et d'un système de ligands comprenant un acide carboxylique et une amine, au moins un dudit acide carboxylique et de ladite amine comportant une chaine hydrocarbonée en C8 à C20.

14. Procédé de préparation selon la revendication 13, selon lequel ledit système de ligands comprend l'acide palmitique et/ou l'hexadécylamine.

15. Procédé de préparation selon la revendication 14, selon lequel ladite étape de carburation est mise en œuvre directement sur les nanoparticules de fer zéro-valent obtenues à l'issue de ladite étape de décomposition.

16. Procédé de préparation selon l'une quelconque des revendications 13 à 15, selon lequel ladite étape de décomposition est mise en œuvre à une température comprise entre 120 à 300 °C.

17. Procédé de préparation selon l'une quelconque des revendications 13 à 16, selon lequel ladite étape de décomposition est mise en œuvre pendant une durée comprise entre 1 et 72 h.

18. Procédé de préparation selon l'une quelconque des revendications 9 à 17, comprenant une étape ultérieure de traitement des nanoparticules de carbure de fer obtenues à l'issue de ladite étape de carburation, par mise en contact desdites nanoparticules de carbure de fer avec un précurseur d'un métal catalytique, de sorte à former un revêtement dudit métal catalytique à la surface desdites nanoparticules de carbure de fer.

19. Utilisation de nanoparticules de carbure de fer selon l'une quelconque des revendications 1 à 8 pour la production de chaleur.

**20.** Utilisation de nanoparticules de carbure de fer selon l'une quelconque des revendications 1 à 8 pour la catalyse de réactions chimiques.

**21.** Utilisation selon la revendication 20, pour la catalyse d'une réaction de réduction de dioxyde de carbone ou de monoxyde de carbone en hydrocarbure(s).

**22.** Procédé de catalyse d'une réaction chimique au moyen de nanoparticules de carbure de fer selon l'une quelconque des revendications 1 à 8, selon lequel lesdites nanoparticules sont introduites dans un milieu réactionnel contenant un ou des réactifs de ladite réaction chimique, et ledit milieu réactionnel est soumis à un champ magnétique apte à provoquer une augmentation de la température desdites nanoparticules jusqu'à une température supérieure ou égale à une température nécessaire à la réalisation de ladite réaction chimique.

**23.** Procédé de catalyse selon la revendication 22, selon lequel le champ magnétique est appliqué à une première amplitude, de préférence supérieure à 50 mT, pendant une première période de temps, de préférence d'une durée comprise entre 3 secondes et 1 minute, puis à une deuxième amplitude, inférieure à ladite première amplitude, de préférence comprise entre 20 et 40 mT, pendant une deuxième période de temps, ladite deuxième période de temps étant plus longue que ladite première période de temps.

**24.** Procédé de catalyse selon l'une quelconque des revendications 22 à 23, selon lequel le champ magnétique est appliqué audit milieu réactionnel de manière puisée.

**25.** Procédé de catalyse selon l'une quelconque des revendications 22 à 24, selon lequel lesdites nanoparticules sont supportées sur un support solide, et ladite réaction chimique réaction est réalisée en flux de réactif(s) continu(s).

**Patentansprüche**

**1.** Eisenkarbidnanopartikel, **dadurch gekennzeichnet, dass** mindestens 70 % der Eisenatome, die er umfasst, in einer kristallinen Struktur von $Fe_{2,2}C$ vorhanden sind.

**2.** Eisenkarbidnanopartikel nach Anspruch 1, **dadurch gekennzeichnet, dass** mindestens 80 % der Eisenatome, die er umfasst, in einer kristallinen Struktur von $Fe_{2,2}C$ vorhanden sind.

**3.** Eisenkarbidnanopartikel nach einem der Ansprüche 1 bis 2 mit einer Größe, die zwischen 1 und 20 nm liegt, die vorzugsweise zwischen 10 und 16 nm liegt.

**4.** Eisenkarbidnanopartikel nach einem der Ansprüche 1 bis 2 mit einer Größe, die gleich 15 nm $\pm$ 1 nm ist.

**5.** Eisenkarbidnanopartikel nach einem der Ansprüche 1 bis 4, der auf mindestens einem Teil seiner Oberfläche mit einer Beschichtung aus einem katalytischen Metall bedeckt ist.

**6.** Eisenkarbidnanopartikel nach Anspruch 5, wobei das katalytische Metall ausgewählt ist aus Nickel, Ruthenium, Kobalt, Kupfer, Zink, Platin, Palladium, Rhodium, Mangan, Molybdän, Wolfram, Vanadium, Iridium, Gold oder einer ihrer Legierungen, alleine oder in Mischung.

**7.** Eisenkarbidnanopartikel nach einem der Ansprüche 1 bis 6, der durch einen Schritt des Vergasens des nullwertigen Eisennanopartikels durch Zusammenbringen des nullwertigen Eisennanopartikels mit einer gasförmigen Mischung aus Dihydrogen und Kohlenstoffmonoxid erhalten werden kann.

**8.** Eisenkarbidnanopartikel nach einem der Ansprüche 1 bis 7, getragen auf einem festen Träger, wobei der feste Träger gegebenenfalls mit einem katalytischen Metall dotiert ist.

**9.** Verfahren zur Herstellung von Eisenkarbidnanopartikel nach einem der Ansprüche 1 bis 8, **dadurch gekennzeichnet, dass** es einen Schritt des Vergasens von nullwertigen Eisennanopartikeln durch Zusammenbringen der nullwertigen Eisennanopartikel mit einer gasförmigen Mischung aus Dihydrogen und Kohlenstoffmonoxid umfasst.

**10.** Verfahren zur Herstellung nach Anspruch 9, wobei der Schritt des Vergasens bei einer Temperatur durchgeführt wird, die zwischen 120 und 300 °C, vorzugsweise zwischen 120 und 180 °C liegt.

**11.** Verfahren zur Herstellung nach einem der Ansprüche 9 bis 10, wobei der Schritt des Vergasens während einer Dauer durchgeführt wird, die zwischen 72 und 200 h liegt.

**12.** Verfahren zur Herstellung nach einem der Ansprüche 9 bis 10, wobei der Schritt des Vergasens die Beseitigung des Wassers umfasst, das nach der Reaktion der nullwertigen Eisennanopartikel und der gasförmigen Mischung im Laufe ihrer Bildung gebildet wird, und der Schritt des Vergasens vorzugsweise während einer Dauer durchgeführt wird, die zwischen 24 und 60 h liegt.

**13.** Verfahren zur Herstellung nach einem der Ansprüche 9 bis 12, umfassend einen vorherigen Schritt des Herstellens der nullwertigen Eisennanopartikel durch Abbau eines organometallischen Vorläufers, der der folgenden allgemeinen Formel (I) entspricht:

$$Fe\ (NR^1R^2)(NR^3R^4) \qquad (I)$$

wobei $R^1$, $R^2$, $R^3$ und $R^4$, identisch oder verschieden, jeweils eine Alkyl-, Aryl-, Trimethylsilyl- oder Trimethylalkyl-Gruppierung
in Anwesenheit von Dihydrogen und eines Systems von Liganden darstellen, die eine Carbonsäure und ein Amin umfassen, wobei mindestens eines der Carbonsäure und des Amins eine Kohlenwasserstoffkette mit C8 bis C20 umfasst.

**14.** Verfahren zur Herstellung nach Anspruch 13, wobei das System von Liganden Palmitinsäure und/oder Hexadecylamin umfasst.

**15.** Verfahren zur Herstellung nach Anspruch 14, wobei der Schritt des Vergasens direkt auf den nullwertigen Eisennanopartikeln ausgeführt wird, die am Ausgang des Schritts des Abbauens erhalten werden.

**16.** Verfahren zur Herstellung nach einem der Ansprüche 13 bis 15, wobei der Schritt des Abbauens bei einer Temperatur durchgeführt wird, die zwischen 120 bis 300 ºC liegt.

**17.** Verfahren zur Herstellung nach einem der Ansprüche 13 bis 16, wobei der Schritt des Abbauens während einer Dauer durchgeführt wird, die zwischen 1 und 72 h liegt.

**18.** Verfahren zur Herstellung nach einem der Ansprüche 9 bis 17, umfassend einen weiteren Schritt des Behandelns der Eisenkarbidnanopartikel, die am Ausgang des Schritts des Vergasens erhalten werden, durch In-Kontakt-Bringen der Eisenkarbidnanopartikel mit einem Vorläufer eines katalytischen Metalls, um eine Beschichtung des katalytischen Metalls auf der Oberfläche der Eisenkarbidnanopartikel zu bilden.

**19.** Verwendung von Eisenkarbidnanopartikeln nach einem der Ansprüche 1 bis 8 zur Erzeugung von Wärme.

**20.** Verwendung von Eisenkarbidnanopartikeln nach einem der Ansprüche 1 bis 8 zur Katalyse von chemischen Reaktionen.

**21.** Verwendung nach Anspruch 20 zur Katalyse einer Reduktionsreaktion von Kohlendioxid oder Kohlenmonoxid in Kohlenwasserstoff(e).

**22.** Verfahren zur Katalyse einer chemischen Reaktion mit Hilfe von Eisenkarbidnanopartikeln nach einem der Ansprüche 1 bis 8, wobei die Nanopartikel in ein Reaktionsmedium eingeführt werden, das eines oder mehrere der Reagenzien der chemischen Reaktion enthält, und das Reaktionsmedium einem magnetischen Feld unterzogen wird, das dazu in der Lage ist, eine Erhöhung der Temperatur der Nanopartikel bis auf eine Temperatur von mehr oder gleich einer Temperatur zu verursachen, die für die Durchführung der chemischen Reaktion erforderlich ist.

**23.** Verfahren zur Katalyse nach Anspruch 22, wobei das magnetische Feld bei einer ersten Amplitude, vorzugsweise höher als 50 mT, während eines ersten Zeitraums angewendet wird, vorzugsweise mit einer Dauer, die zwischen 3 Sekunden und 1 Minute liegt, dann einer zweiten Amplitude, die kleiner als die erste Amplitude ist, die vorzugsweise zwischen 20 und 40 mT liegt, während eines zweiten Zeitraums, wobei der zweite Zeitraum länger als der erste Zeitraum ist.

**24.** Verfahren zur Katalyse nach einem der Ansprüche 22 bis 23, wobei das magnetische Feld auf das Reaktionsmedium

auf gepulste Weise angewendet wird.

**25.** Verfahren zur Katalyse nach einem der Ansprüche 22 bis 24, wobei die Nanopartikel von einem festen Träger getragen werden und die chemische Reaktion im Fluss von kontinuierlichem Reagens/kontinuierlichen Reagenzien durchgeführt wird.

## Claims

**1.** Iron carbide nanoparticle, **characterized in that** at least 70% of the iron atoms that it comprises are present in an $Fe_{2.2}C$ crystalline structure.

**2.** Iron carbide nanoparticle as claimed in claim 1, **characterized in that** at least 80% of the iron atoms that it comprises are present in an $Fe_{2.2}C$ crystalline structure.

**3.** Iron carbide nanoparticle as claimed in either one of claims 1 and 2, having a size of between 1 and 20 nm, preferably between 10 and 16 nm.

**4.** Iron carbide nanoparticle as claimed in either one of claims 1 and 2, having a size equal to 15 nm $\pm$ 1 nm.

**5.** Iron carbide nanoparticle as claimed in any one of claims 1 to 4, covered on at least part of its surface with a coating of a catalytic metal.

**6.** Iron carbide nanoparticle as claimed in claim 5, wherein said catalytic metal is chosen from nickel, ruthenium, cobalt, copper, zinc, platinum, palladium, rhodium, manganese, molybdenum, tungsten, vanadium, iridium, gold, or any one of the alloys thereof, alone or as a mixture.

**7.** Iron carbide nanoparticle as claimed in any one of claims 1 to 6, obtainable by means of a step of carburization of a zero-valent iron nanoparticle by bringing said zero-valent iron nanoparticle into contact with a gas mixture of dihydrogen and carbon monoxide.

**8.** Iron carbide nanoparticle as claimed in any one of claims 1 to 7, supported on a solid support, said solid support being optionally doped with a catalytic metal.

**9.** A preparation method for preparing iron carbide nanoparticles as claimed in any one of claims 1 to 8, **characterized in that** it comprises a step of carburization of zero-valent iron nanoparticles by bringing said zero-valent iron nanoparticles into contact with a gas mixture of dihydrogen and carbon monoxide.

**10.** The preparation method as claimed in claim 9, wherein said carburization step is carried out at a temperature of between 120 and 300°C, preferably between 120 and 180°C.

**11.** The preparation method as claimed in either one of claims 9 and 10, wherein said carburization step is carried out for a period of between 72 and 200 h.

**12.** The preparation method as claimed in either one of claims 9 and 10, wherein said carburization step comprises the removal of the water formed during the reaction of said zero-valent iron nanoparticles and of said gas mixture, as said water is formed, and said carburization step is preferably carried out for a period of between 24 and 60 h.

**13.** The preparation method as claimed in any one of claims 9 to 12, comprising a prior step of preparing the zero-valent iron nanoparticles by decomposition of an organometallic precursor corresponding to general formula (I):

$$Fe(NR^1R^2)(NR^3R^4) \qquad (I)$$

wherein $R^1$, $R^2$, $R^3$ and $R^4$, which may be identical or different, each represent an alkyl, aryl, trimethylsilyl or trimethylalkyl group,
in the presence of dihydrogen and of a ligand system comprising a carboxylic acid and an amine, at least one of said carboxylic acid and of said amine comprising a $C_8$ to $C_{20}$ hydrocarbon-based chain.

**14.** The preparation method as claimed in claim 13, wherein said ligand system comprises palmitic acid and/or hexadecylamine.

**15.** The preparation method as claimed in claim 14, wherein said carburization step is carried out directly on the zero-valent iron nanoparticles obtained at the end of said decomposition step.

**16.** The preparation method as claimed in any one of claims 13 to 15, wherein said decomposition step is carried out at a temperature of between 120 and 300°C.

**17.** The preparation method as claimed in any one of claims 13 to 16, wherein said decomposition step is carried out for a period of between 1 and 72 h.

**18.** The preparation method as claimed in any one of claims 9 to 17, comprising a subsequent step of treating the iron carbide nanoparticles obtained at the end of said carburization step, by bringing said iron carbide nanoparticles into contact with a precursor of a catalytic metal, so as to form a coating of said catalytic metal at the surface of said iron carbide nanoparticles.

**19.** The use of iron carbide nanoparticles as claimed in any one of claims 1 to 8, for heat production.

**20.** The use of iron carbide nanoparticles as claimed in any one of claims 1 to 8, for the catalysis of chemical reactions.

**21.** The use as claimed in claim 20, for the catalysis of a reaction for reduction of carbon dioxide or of carbon monoxide into hydrocarbon(s).

**22.** A catalysis method for catalyzing a chemical reaction by means of iron carbide nanoparticles as claimed in any one of claims 1 to 8, wherein said nanoparticles are introduced into a reaction medium containing one or more reagents for said chemical reaction, and said reaction medium is subjected to a magnetic field capable of causing an increase in the temperature of said nanoparticles up to a temperature of greater than or equal to a temperature required for carrying out said chemical reaction.

**23.** The catalysis method as claimed in claim 22, wherein the magnetic field is applied at a first amplitude, preferably of greater than 50 mT, for a first period of time, preferably for a period of between 3 seconds and 1 minute, then at a second amplitude, of less than said first amplitude, preferably of between 20 and 40 mT, for a second period of time, said second period of time being longer than said first period of time.

**24.** The catalysis method as claimed in either one of claims 22 and 23, wherein the magnetic field is applied to said reaction medium in a pulsed manner.

**25.** The catalysis method as claimed in any one of claims 22 to 24, wherein said nanoparticles are supported on a solid support, and said chemical reaction is carried out in a flow of continuous reagent(s).

FIG 1

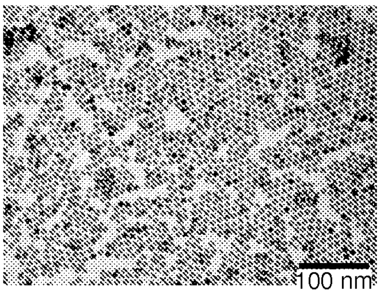


(a)

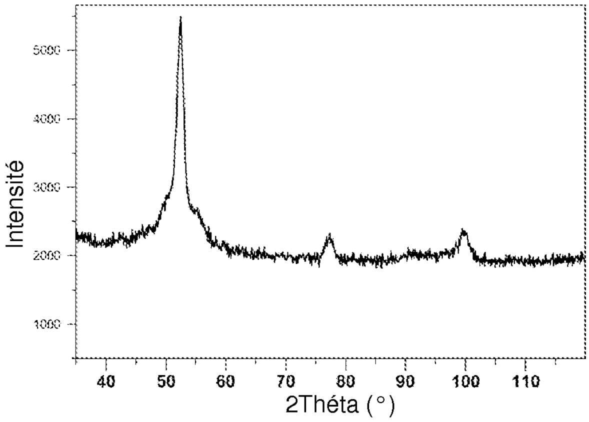


(b)

FIG 2

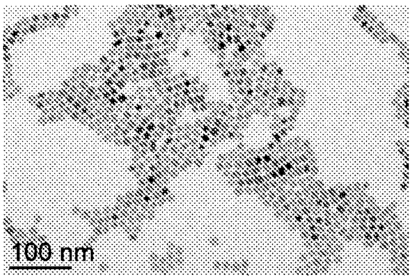


(a)

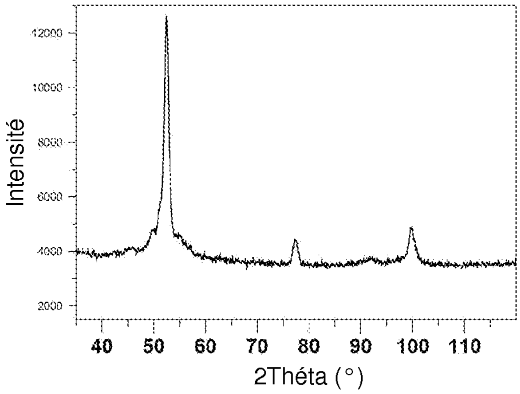


(b)

FIG 3

(a)

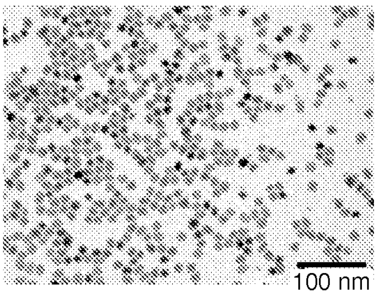


(b)

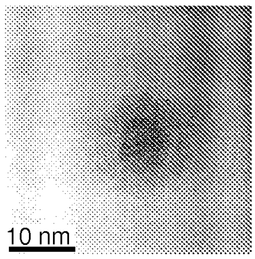


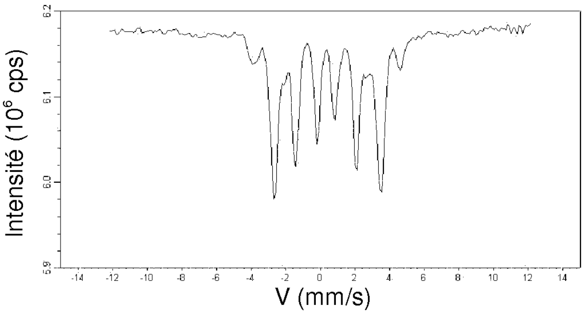


(d)

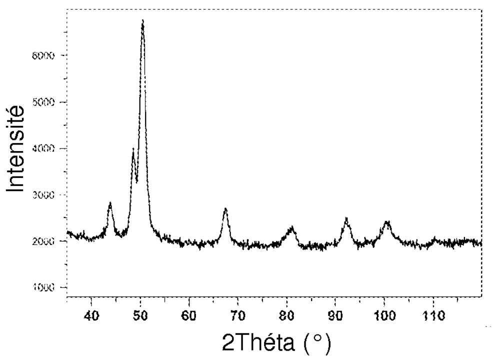


(c)

FIG 5

(a)

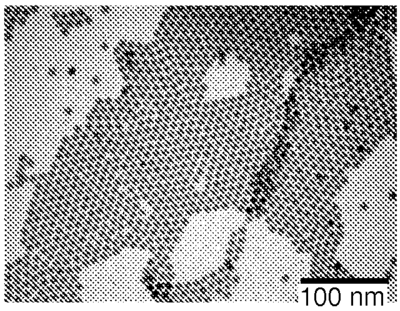


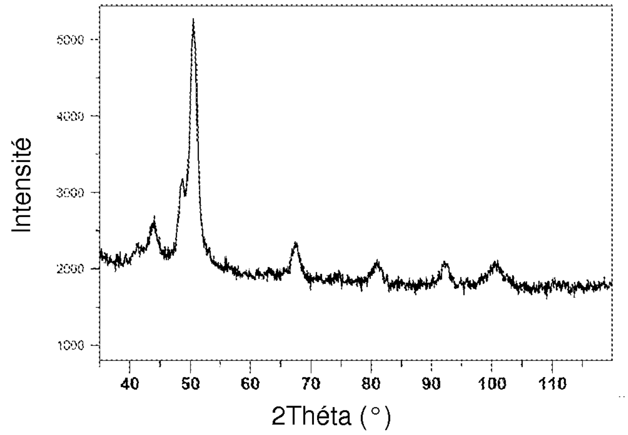


(b)

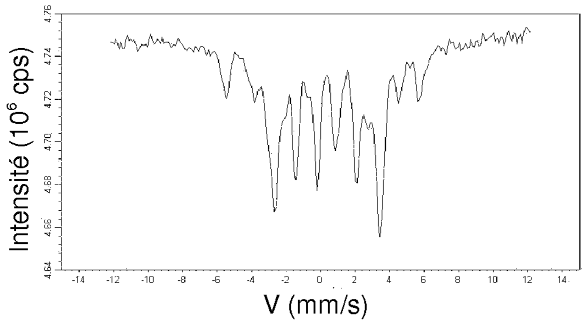


(c)

FIG 4

(a)

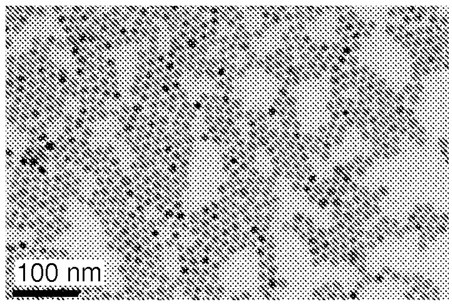


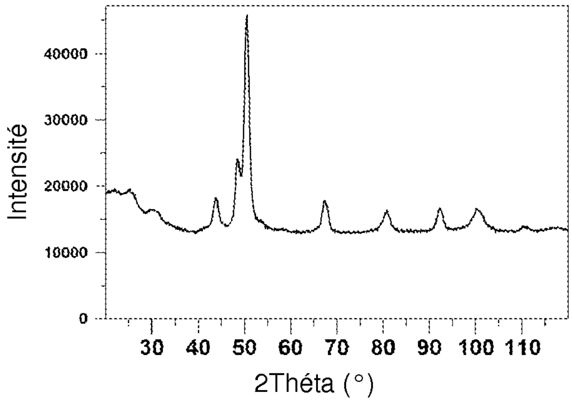


(b)

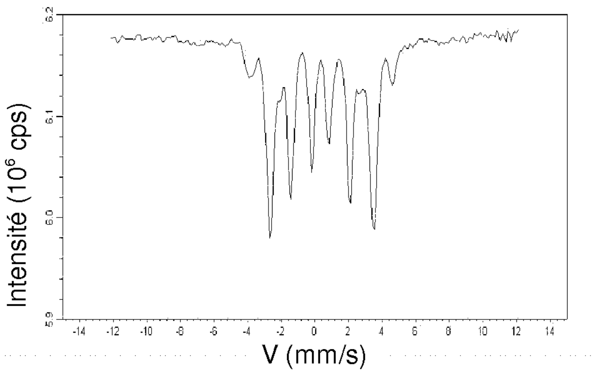


(c)

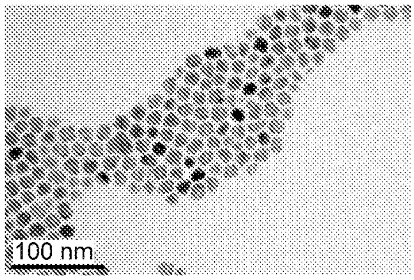


(a)

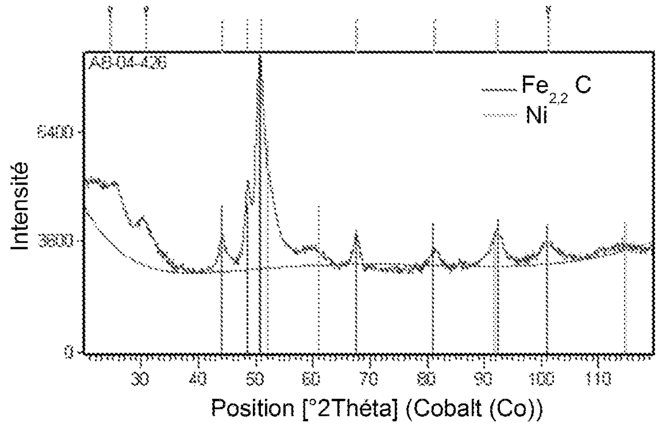


FIG 6

(b)

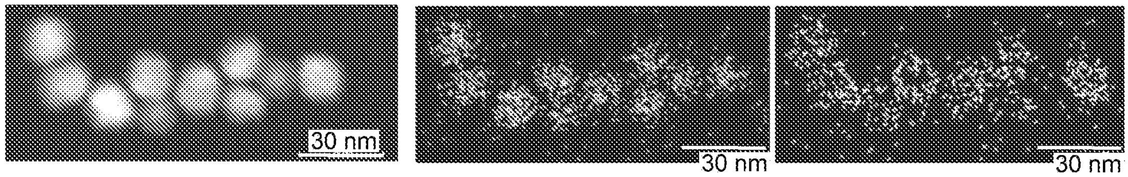


(c1) (c2) (c3)

FIG 7

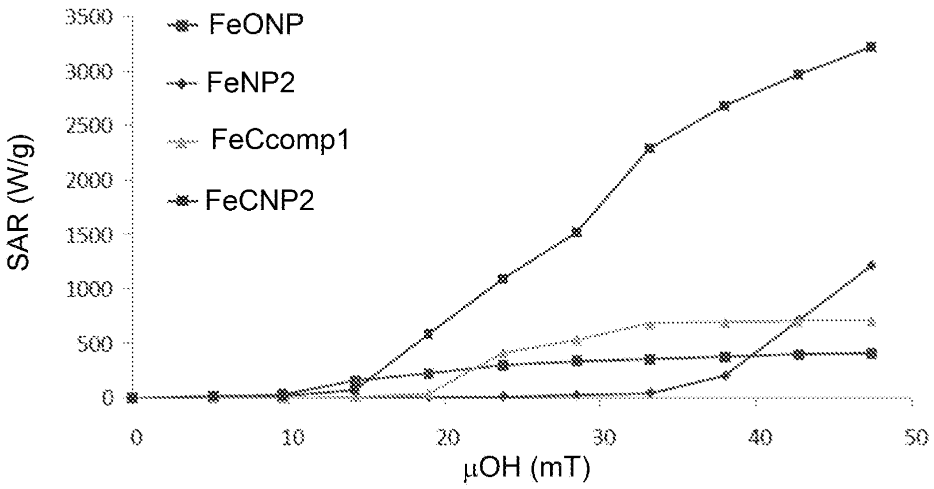


FIG 8

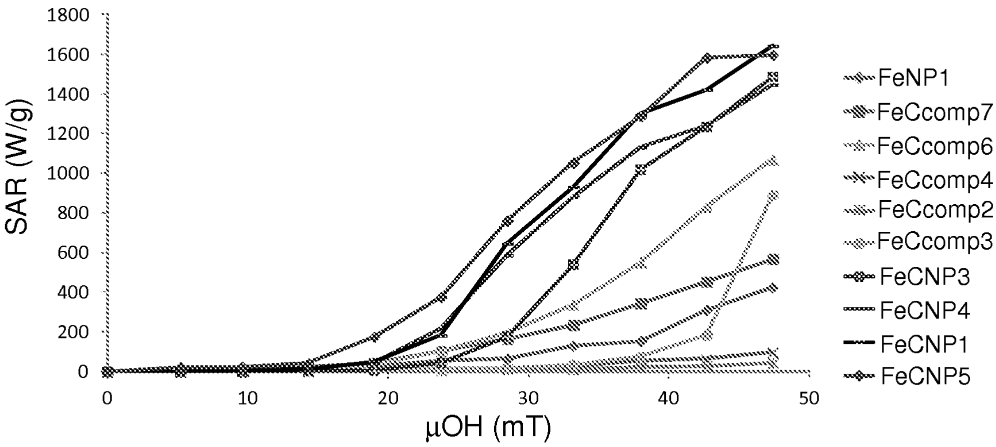

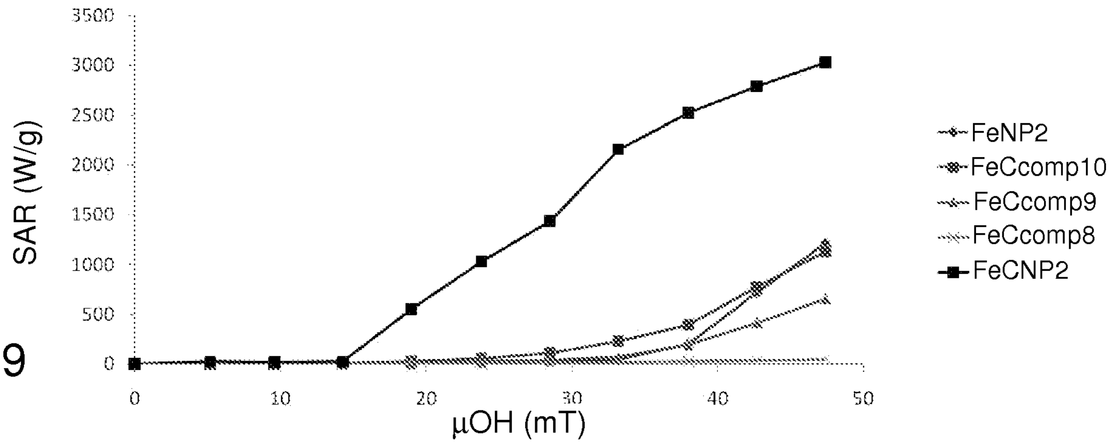


FIG 9

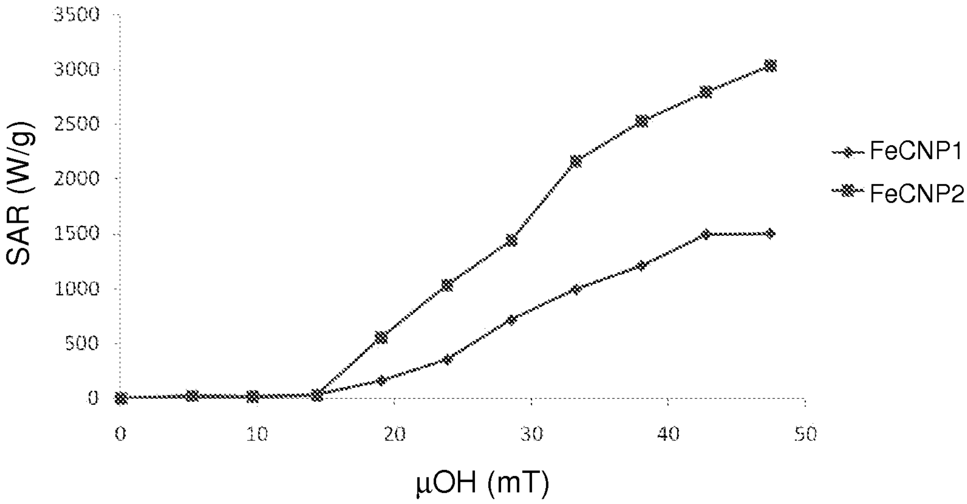


FIG 10

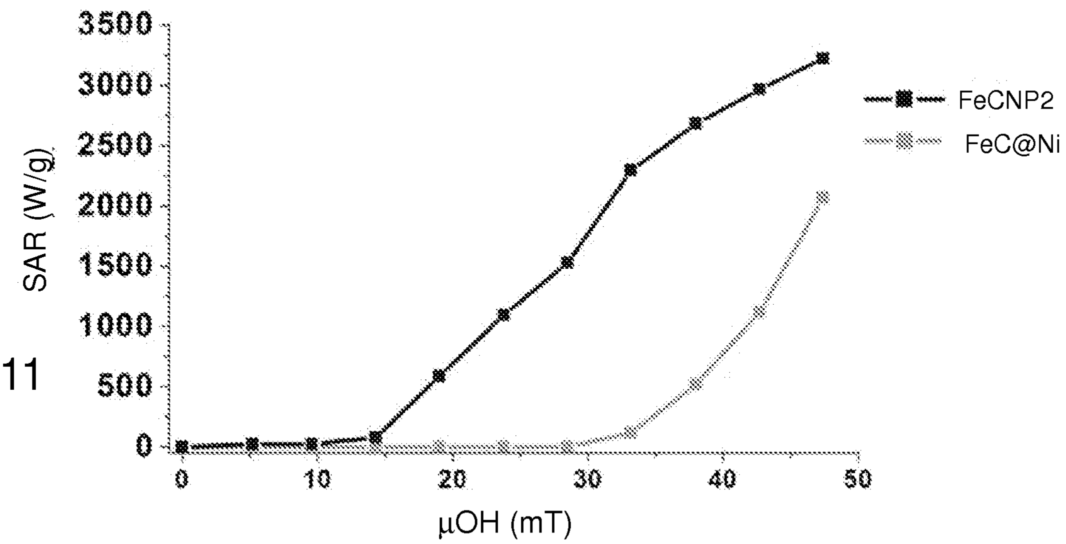


FIG 11

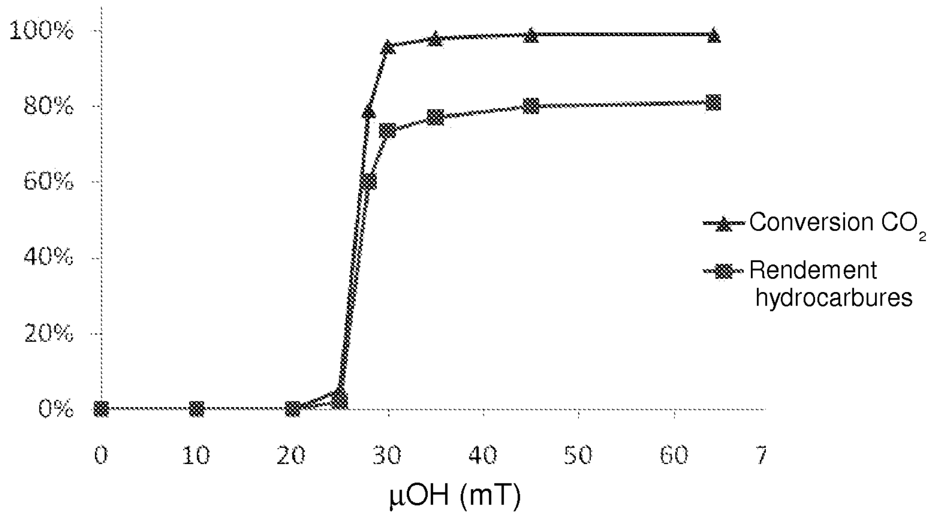


FIG 12

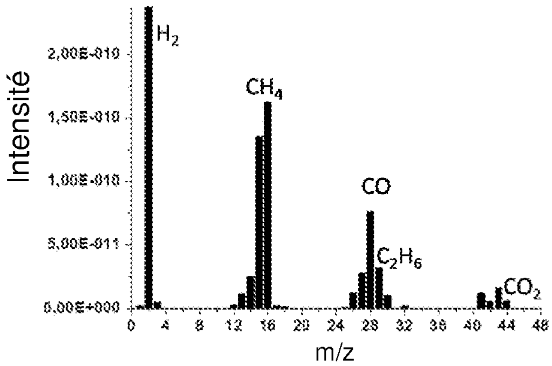


FIG 13

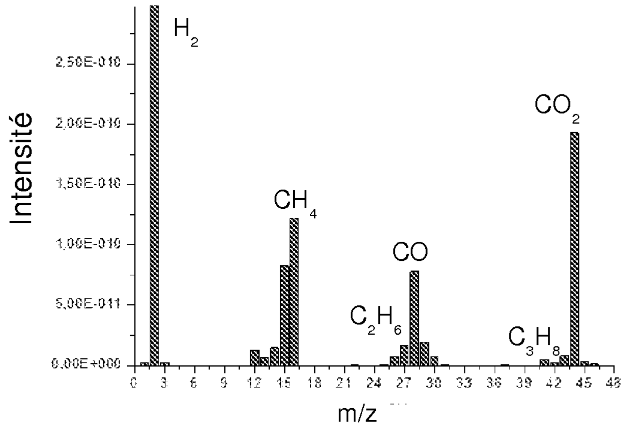


FIG 14

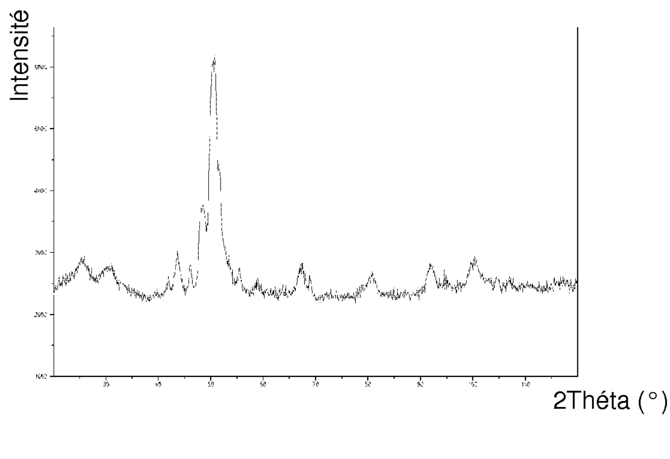


FIG 15

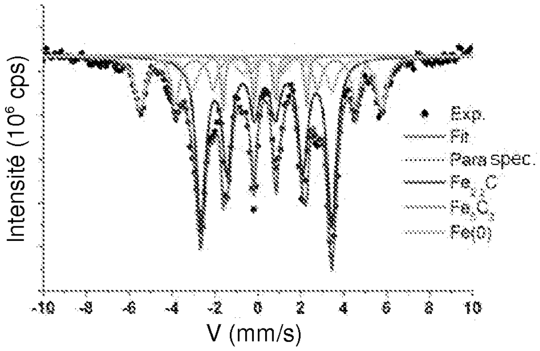


a/

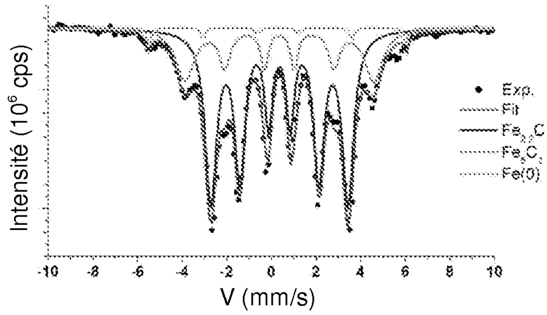


b/

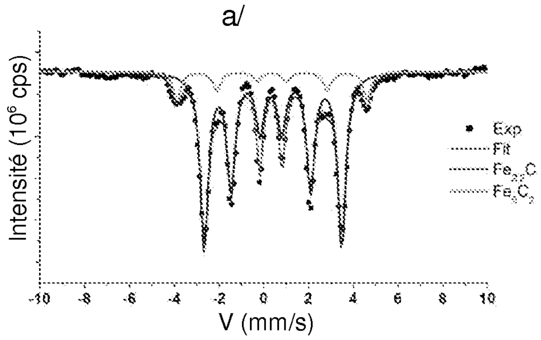


c/

FIG 16

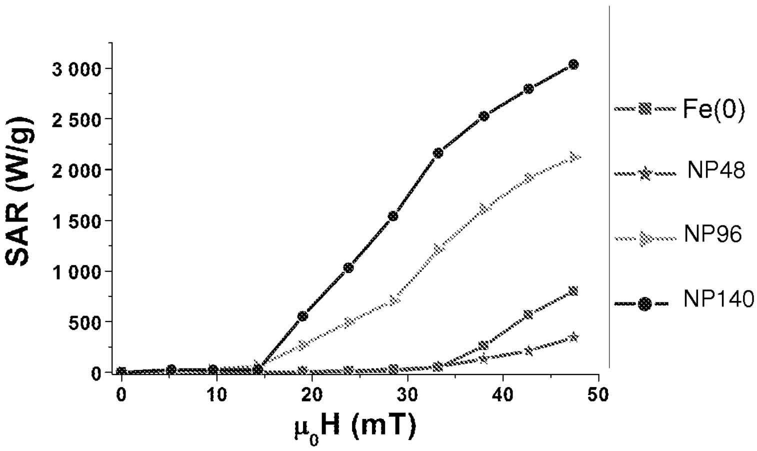


FIG 17

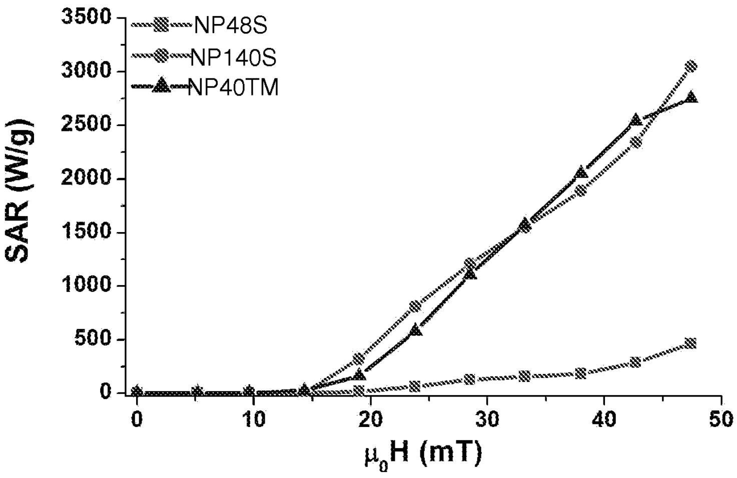


FIG 18

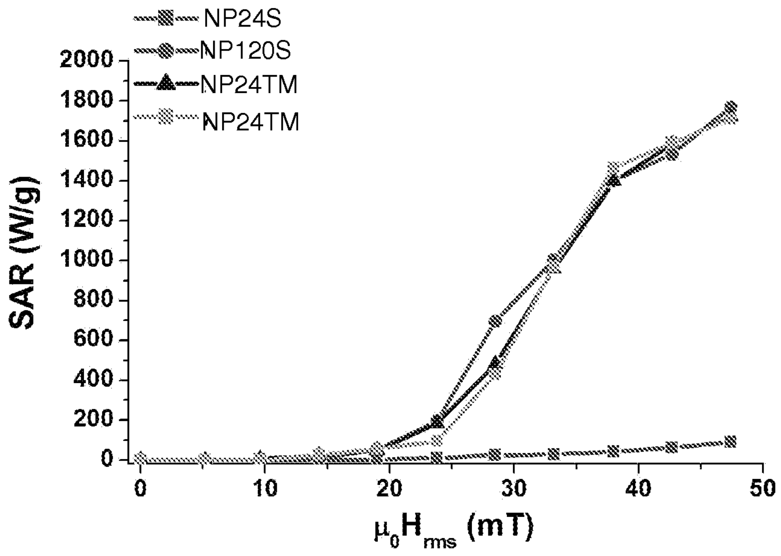


FIG 19

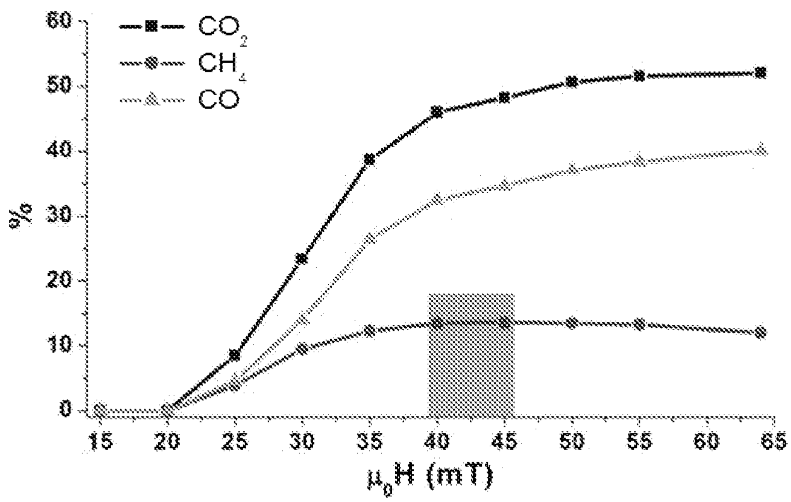


FIG 20

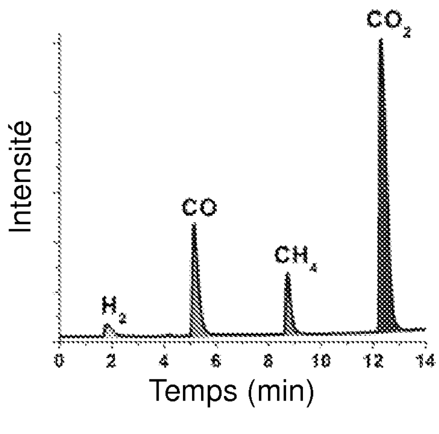


FIG 21

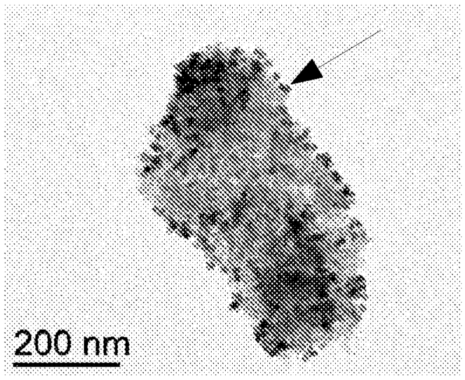


FIG 22

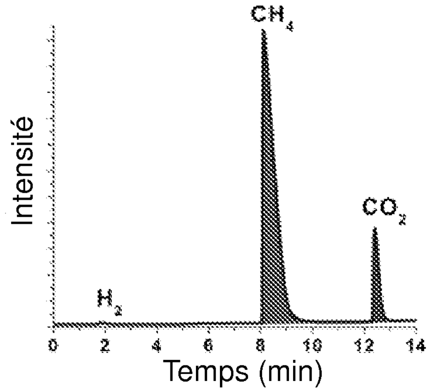


FIG 24

FIG 23

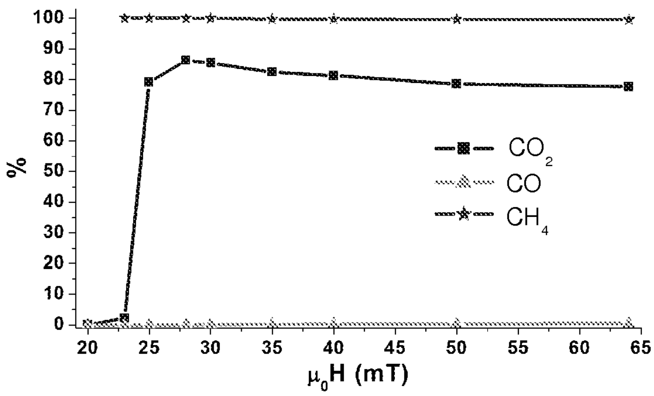


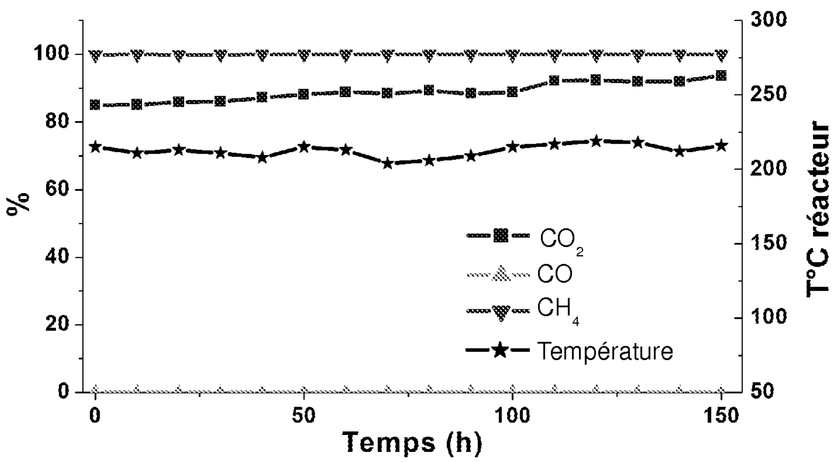


FIG 25

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014162099 A


### Littérature non-brevet citée dans la description


- **YANG et al.** *J. Am. Chem. Soc.,* 2012, vol. 134, 15814-15821
- **MEFFRE et al.** *Nano Letters,* 2012, vol. 12, 4722-4728
- **LIU et al.** *Nanotechnology,* 2015, vol. 26, 085601
- *CHEMICAL ABSTRACTS,* 1318-02-1
- **MEFFRE et al.** *Nanoletters,* 2012, 4722-4728
- **PELLEGRINO et al.** *J. Mater. Chem. B,* 2014, 4426-4434
- **MEFFRE et al.** *Nanoletters,* 2012, vol. 12, 4722-4728